(19) Europäisches Patentamt
European Patent Office
Office européen des brevets

(11) EP 0 454 078 B1

(12) **EUROPEAN PATENT SPECIFICATION**

(45) Date of publication and mention of the grant of the patent:
**02.10.1996 Bulletin 1996/40**

(51) Int. Cl.[6]: **A61K 49/00**, A61K 51/00

(21) Application number: **91106575.3**

(22) Date of filing: **24.04.1991**

(54) **Dual functioning excipient for metal chelate contrast agents**

Dual-Funktionnierende Excipient für Metalchelate-Kontrastmitteln

Excipient fonctionnant de manière duale pour les agents de contraste contenant un chélate métallique

(84) Designated Contracting States:
**AT BE CH DE DK ES FR GB GR IT LI LU NL SE**

(30) Priority: **25.04.1990 US 514468**

(43) Date of publication of application:
**30.10.1991 Bulletin 1991/44**

(73) Proprietor: **BRACCO INTERNATIONAL B.V.**
**1083 HJ Amsterdam (NL)**

(72) Inventors:
- **Chang, C. Allen**
  **Robbinsville, NJ (US)**
- **Kumar, Krishan**
  **East Windsor, NJ (US)**
- **Tweedle, Michael F.**
  **Princeton, NJ (US)**

(74) Representative: **Chopard, Pierre-Antoine et al**
**c/o BRACCO Research S.A.**
**7, Route de Drize**
**1227 Carouge (CH)**

(56) References cited:
**EP-A- 0 232 751**
**EP-A- 0 255 471**
**EP-A- 0 270 483**
**EP-A- 0 292 689**
**WO-A-89/00052**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 454 078 B1

## Description

The use of metal chelates as contrast agents for magnetic resonance, x-ray, ultrasound, and radiodiagnostic imaging is well known. Metal chelates of the transition metal and lanthanide metal ions are of primary interest due to their strong paramagnetic and x-ray absorbing properties as well as others. Because the free metal ions are in general more toxic, these type of pharmaceuticals are prepared in the form of chelates, i.e. the metal ions are complexed, typically by organic ligands. Examples of these organic ligands are linear and macrocyclic polyaminopolycarboxylic acids and their derivatives. Unfortunately, in many cases there are also toxicity problems with the free organic ligand. Thus, even the use of metal chelates as contrast agents may cause toxicity problems to the extent that free metal and/or free organic ligand may both be present in the blood following introduction of the chelate.

The European patent 0270483 discloses the use of a formulation excipient of the formula

$$M_x[M'(L)]_y$$

which is described as the metal (M = e.g. sodium) salt of a less toxic metal (M' = e.g. Zinc, copper or calcium) chelate, wherein L may or may not be the organic ligand with which the paramagnetic or heavy metal is complexed. This excipient is disclosed as a scavenger for the free metal ions, but no mention is made of scavenging free organic ligand. Indeed, this European patent suggests and claims the <u>addition</u> of free ligand to enhance safety.

In WO 89/00052 entitled "Metal-Ligand Chelates Safety Enhancement - Used in Magnetic Resonance Imaging or X-ray Contrast Agents, by Addition of Calcium Ions", it is claimed that the use of effective amounts of calcium in the form of, calcium chloride, calcium gluconate, or balanced salt solutions substantially reduces the toxicity without the need to add additional ligand. The potential toxicity from free metal ion and/or organic ligand was not discussed.

In accordance with the present invention novel excipients for metal chelate contrast agents, M(L), wherein M is a contrast metal and L is a chelating ligand, for magnetic resonance, x-ray, ultrasound and radiodiagnostic imaging, and compositions and methods utilizing such excipients, are disclosed. These novel excipients have the formula

$$X_m[X'(L')]_n , \qquad (I)$$

wherein X and X' are each independently calcium or zinc;

L' is an organic ligand which may be the organic ligand, L, employed in the metal chelate contrast agent or another organic ligand which has a greater affinity for the metal, M, than for calcium or zinc; and wherein m and n are independently selected from 1, 2 or 3. This salt of the complex of the organic ligand is a highly useful excipient for metal chelate contrast agents in that this single excipient has been found to scavenge free metal ions and free organic ligand, thereby enhancing the safety of such contrast agents and methods employing same.

The present invention pertains to novel excipients for metal chelate contrast agents, compositions of contrast agents complexed with such excipients and methods of imaging employing same. Unexpectedly, the novel excipient of the present invention, comprising the salt of the complex of an organic ligand, has been found to scavenge free metal and free organic ligand. Preferably, the excipient comprises the calcium salt of the calcium complex of the ligand shown as

$$Ca_m[Ca(L')]_n$$

wherein m, n and L' are as defined above. Since the excipients of the present invention are dual-functioning scavengers and are much safer than the free metal ions and free ligands they scavenge, significantly less toxic contrast agents and methods of imaging are provided.

This dual-functioning phenomenon is additionally advantageous in that the possible toxicity resulting from any dissociation of a metal chelate contrast agent, M(L), which may occur while in storage prior to use is also alleviated. Thus, products with enhanced safety and shelf-life are provided by use of the present excipients.

In viewing the dual scavenging activity of the present excipient

$$X_m[X'(L')]_n ,$$

it is believed that the calcium or zinc complex, X'(L'), reacts with the toxic metal ion of the contrast agent by a metal ion exchange process. The free calcium or zinc ion of the excipient forms a complex with free organic ligand that may be present. No other new species are expected to form *in situ* utilizing this excipient. The calcium or zinc complex salts of formula I are readily prepared by reacting the desired organic ligand in solution with an excess of a calcium salt, e.g., calcium carbonate, calcium chloride, calcium acetate, zinc chloride, zinc acetate and the like.

Thereafter, the present excipients can be employed in compositions with any metal chelate contrast agent comprising a metal ion and an organic ligand. This can be accomplished, using known techniques, by adding the calcium or

zinc complex salt of formula I to a solution of the metal chelate contrast agent, as more clearly illustrated in the Examples.

As would be understood by those working in this art, the organic ligand, L', should be selected so that the complexes it forms, i.e., Ca(L'), Zn(L') and/or M(L') are well tolerated. Suitable organic ligands include but are not limited to linear or macrocyclic polyaminopolycarboxylic acids and derivatives thereof. One preferred group metal chelate contrast agents and imaging methods of the present invention employ organic ligands which are 1-substituted-1,4,7-triscarboxymethyl-1,4,7,10-tetraazacyclododecane and derivatives thereof, as disclosed in U. S. 4,885,363 and pending applications U. S. Ser. No. 454,883, filed December 22, 1989 entitled "10-(2'-Hydroxy-3'-Alkoxy-1,4,7-Triscarboxymethyl-1,4,7,10-Tetraazacyclododecanes" and U. S. Ser. No. 454,890, filed December 22, 1989 entitled 10-(2'-Hydroxy-3'-Polyoxaalkyl)-1,4,7-Triscarboxymethyl-1,4,7,10-Tetraazacyclododecane", which have the general formula

A

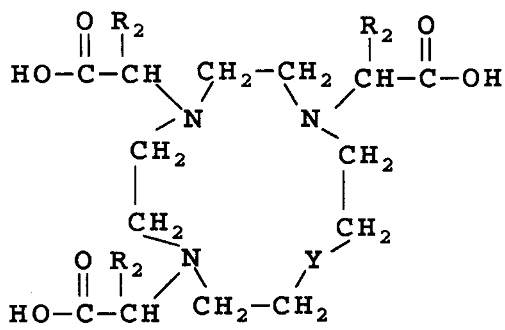 ;

where Y is oxygen or

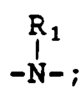

$R_1$ and $R_2$ are each independently hydrogen, alkyl, arylalkyl, aryl, alkoxy, hydroxyalkyl, hydroxyalkoxy

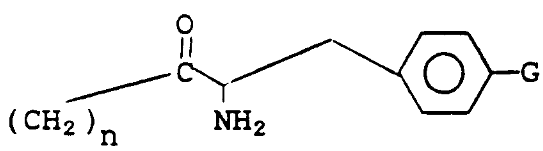 , 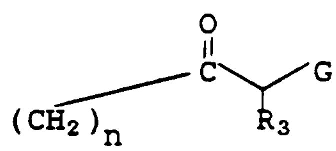

$-(CH_2)_nG$,

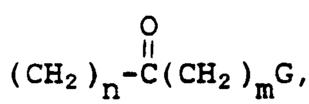 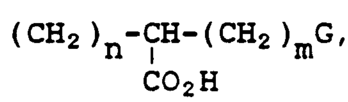

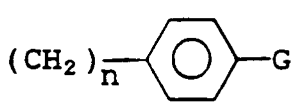 ,

wherein G is $NH_2$, NCS,

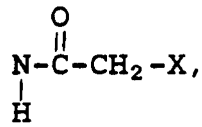

$CO_2H$, $NHR_4$, $N(R_4)_2$, CN, wherein $R_4$ is alkyl or hydroxyalkyl, hydroxyalkoxy,

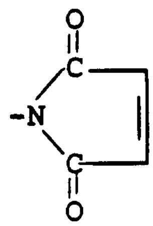 , 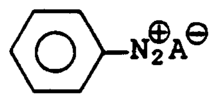

(where A is an anion),

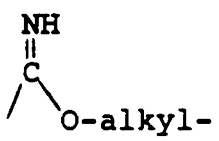 , 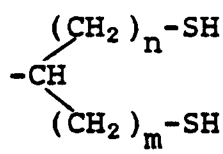 ,

wherein n and m are zero or an integer from one to five, $R_3$ is hydrogen, hydroxyalkyl, alkoxy, alkyl, aryl, arylalkyl or hydroxyalkoxy and X is chloro, bromo or iodo.

$R_1$ and $R_2$ are hydrogen in a preferred embodiment for forming a Gd(III) chelate useful in general purpose magnetic resonance imaging. The most preferred emobodiment for forming a Gd(III) chelate is when $R_1$ is hydroxyalkyl or when $R_1$ is

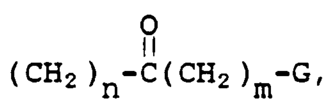

wherein n is 1, m is 0, G is $NHR_4$ wherein $R_4$ is alkyl. Thus, preferred ligands from this group are 1,4,7,10-tetraazacyclododecane-1,4,7-triacetic acid, i.e., DO3A, 1,4,7-tris(carboxymethyl)-10-(2'-hydroxypropyl)-1,4,7,10-tetraazacyclododecane, i.e., HP-DO3A, and 1,4,7-tris(carboxymethyl)-1,4,7,10-tetraazacyclododecane.

Another group of suitable ligands are disclosed in U. S. 4,647,447 which describes the complex salts

B

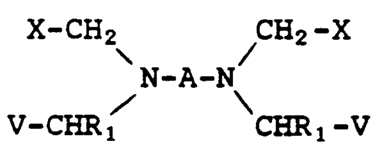

or

C

$$N(CH_2X)_3$$

wherein

X is -COOY, $PO_3HY$ or -CONHOY;

Y is a hydrogen atom, a metal ion equivalent and/or a physiologically biocompatible cation of an inorganic or organic base or amino acid;

A is $-CHR_2-CHR_3-$, $-CH_2CH_2(ZCH_2-CH_2)_m-$,

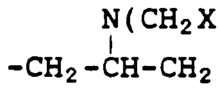 , or 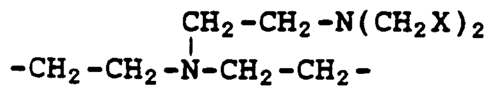 ,

wherein X is as defined above;

each $R_1$ is hydrogen or methyl;

$R_2$ and $R_3$ together represent a trimethylene group or a tetramethylene group or individually are hydrogen atoms, lower alkyl groups (e.g., 1-8 carbons), phenyl groups, benzyl groups or $R_2$ is a hydrogen atom and $R_3$ is $-(CH_2)_p-C_6H_4$-W-protein where p is 0 or 1, W is -NH-, $-NHCOCH_2-$ or -NHCS-, protein represents a protein residue;

m is 1, 2 or 3;

Z is an oxygen atom or a sulfur atom or the group $NCH_2X$ or $NCH_2CH_2OR_4$ wherein X is as defined above and $R_4$ is $C_{1-8}$alkyl;

V is X or is $-CH_2OH$, $-CONH(CH_2)_nX$ or -COB, wherein X is as defined above, B is a protein or lipid residue, n is an integer from 1 to 12, or if $R_1$, $R_2$ and $R_3$ are each hydrogen; then both V's together form the group

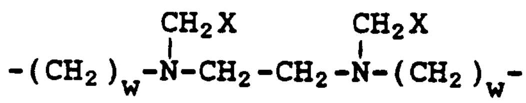

where X is as above, w is 1, 2 or 3, provided that at least two of the substituents Y represent metal ion equivalents of an element with an atomic number of 21 to 29, 42, 44 or 57 to 83. Preferred ligands from U. S. 4,647,447 include 1,4,7,10-tetraazacyclododecane-N,N'N",N'"-tetraacetic acid, i.e., DOTA, and diethylene triamine pentaacetic acid, i.e., DTPA.

Also related to the U. S. 4,647,447 ligands are diethylenetriamine pentaacetic acid-bis methylamide (DTPA-BMA), which can be shown as

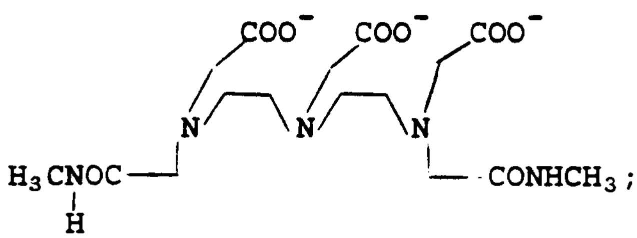

DTPA-bis morpholino amide ((a) below); and DTPA-bis 1,2-dihydroxypropylamide ((b) below)

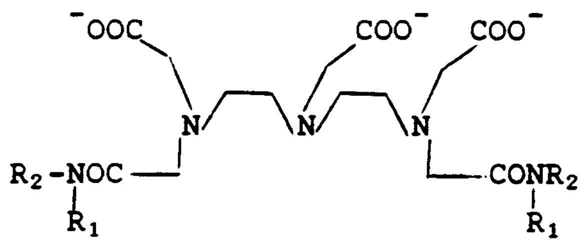

a) $R_1$ and $R_2$ together with nitrogen to which they are attached form morpholino,
b) $R_1$ is hydrogen and $R_2$ is

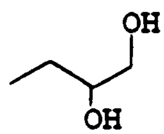

.

Another group of ligands suitable for use with the present excipients/compositions/methods is disclosed in European Application 0 255 471 A1. That application discloses macrocycles of the formula

D

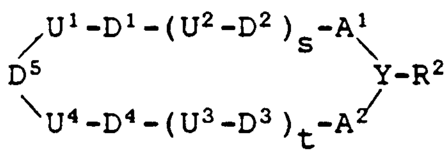

wherein

Y is N or P;

$A^1$ and $A^2$ are each optionally branched $C_{2-6}$ alkylene;

$U^1$, $U^2$, $U^3$ and $U^4$ are each a single bond or optionally branched $C_{1-6}$ alkylene;

$D^1$, $D^2$, $D^3$, $D^4$ are each O, S, $C_{1-6}$ alkylene or $NR_7$;

$R_7$ is hydrogen or $C_{1-4}$ alkylene having a $COOR^1$ terminal group;

$R^1$ is hydrogen or a metal ion equivalent;

$D^5$ is $D^1$ or $CHR^5$, where $R^5$ can be hydrogen or optionally unsaturated $C_{1-20}$ alkylene which may include imino, phenyleneoxy, phenyleneimino, amido, ester, O, S and/or N optionally substituted with OH, SH imino and/or amino and may carry a terminal functional group (optionally bonded to a macromolecule B);

s and t are each 0-5;

$R_2$ is hydrogen, optionally substituted $C_{1-16}$ alkyl, acyl, acylalkyl (optionally substituted by one or more OH or lower alkoxy groups), $-CH_2-X-V$, B or $CH_2COB$ where X is CO, optionally branched $C_{1-10}$ alkylene (optionally substituted by 1 or more OH or lower alkoxy groups) or optionally branched $C_{2-23}$ alkylene interrupted by O;

V is $NR^3R^4$ or $COOR^6$;

$R^3$ and $R^4$ are each hydrogen, $C_{1-16}$ alkyl (optionally substituted by 1 or more OH or lower alkoxy groups) or together complete a 5-6 membered heterocycle optionally containing another heteroatom;

$R_6$ is hydrogen, $C_{1-16}$ saturated, unsaturated, linear branched or cyclic hydrocarbyl, aryl or aralkyl;

$R_2$ or $R_3$ can be bonded by a $C_{2-20}$ alkylene chain (optionally having a terminal carbonyl group, optionally

interrupted by 1 or more O or $R^1$ carboxymethylimino, or substituted by one or more OH, lower alkoxy or carboxy lower alkyl groups) to a second macromolecule of the formula

D'

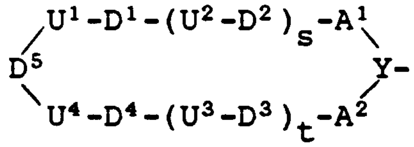

which second macromolecule D' can be the same as or different from the macromolecule of D.

As described above, the excipients of the present invention can be employed with any metal chelate contrast agent. In one embodiment they are used with a contrast agent comprising an organic ligand of formula A complexed with a paramagnetic metal atom and used as relaxation enhancement agents for magnetic resonance imaging. These agents, when administered to a mammalian host (e.g., humans) distribute in various concentrations to different tissues, and catalyse relaxation of protons (in the tissues) that have been excited by the absorption of radiofrequency energy from a magnetic resonance imager. This acceleration of the rate of relaxation of the excited protons provides for an image of different contrast when the host is scanned with a magnetic resonance imager. The magnetic resonance imager is used to record images at various times generally before and after administration of the agents, and the differences in the images created by the agents' presence in tissues are used in diagnosis. In proton magnetic resonance imaging, paramagnetic metal atoms such as gadolinium(III), dysprosium(III), manganese(II), manganese(III) chromium(III), iron(II) and iron(III) (all are paramagnetic metal atoms with a symmetrical electronic configuration) are preferred as metals complexed by the ligands of formula I; gadolinium(III) is most preferred due to the fact that it has the highest paramagnetism, low toxicity, and high lability of coordinated water.

Exemplary contrast agents which will be greatly enhanced when employed in a composition including a pharmaceutically acceptable carrier and an excipient of the present invention include Gadoteridol which is Gd(HP-DO3A), Dotarem which is N-methylglucamine[Gd(DOTA)], Magnevist which is di-N-methylglucamine[Gd(DTPA)] and Gadodiamide which is Gd(DTPA-BMA), Gd(DTPA)-bis morpholinoamide, Gd(DTPA)-bis 1,2-dihydroxypropylamide.

Additionally, the excipients of formula I are conveniently employed with ligands of formula A which are complexed with a lanthanide (atomic number 58 to 71) and used as chemical shift agents in magnetic resonance imaging or in magnetic resonance *in vivo* spectroscopy.

Excipients of formula I are also conveniently employed with contrast elements including yttrium and the transition series (atomic number 21-29).

While the above-described uses for the excipients of formula I and contrast agents including same are preferred, those working in the medical diagnostic arts will appreciate that the excipients can also be used with contrast agents in x-ray imaging, radionuclide imaging and ultrasound imaging.

As mentioned previously excipients, compositions and methods wherein X = X' = calcium are preferred and these excipients, compositions and methods where L' = L are most preferred.

As described previously, contrast agents which include the excipient of formula I can be easily prepared by adding the calcium or zinc complex salt into the metal chelate contrast agent solution. Preferably the so-formed solution is maintained at about pH neutral. The excipient/contrast agent composition can be prepackaged in combination or the present excipient can be added to the contrast solution directly before use. Typically the mole ratio of the calcium or zinc complex salt to the contrast agent is about 0.05-10 percent.

The following Examples illustrate specific embodiments of the present invention, however, it is understood that the invention should not be limited to the details therein.

## Example 1

Calcium bis[1,4,7,10-tetraazacyclododecane-1,4,7-triacetatocalcium(II)], Ca[Ca(DO3A)]$_2$*

Twenty millimoles (7.67 g) of DO3A was dissolved in 32 mL of water and 32 mmoles (3.18 g) of calcium carbonate was added slowly. The solution was heated under reflux at 80°C for 2 hours and it was cooled to room temperature and filtered to remove excess solid calcium carbonate. The solution was then rotary evaporated to dryness and placed in a

*DO3A = 1,4,7,10-tetraazacyclododecane-1,4,7-triacetate

75°C vacuum oven (5 mm Hg) for 24 hours. A glass-like solid was obtained in 94 percent yield.

| Analysis calc'd $Ca_3C_{28}H_{46}N_8O_{12}$ (8% $H_2O$): | | | |
|---|---|---|---|
| | C, 38.58; | H, 6.15; | N, 12.85; |
| Found: | C, 38.17; | H, 5.99; | N, 12.83. |

Example 2

Calcium bis[1,4,7-tris(carboxymethyl)-10-(2'-hydroxy)propyl-1,4,7,10-tetraazacyclododecanatocalcium(II)], $Ca[Ca(HP\text{-}DO3A)]_2$**

The ligand HP-DO3A, containing 3.5 percent water by elemental analysis data, (0.518 g, 1.24 mmol) was dissolved in 10 mL water at room temperature and solid calcium carbonate (0.204 g, 2.03 mmol) was added to it slowly with stirring. The cloudy solution was heated at 90°C for 2.5 hours, cooled, centrifuged, and filtered. The filtrate was evaporated under vacuum to dryness to give 0.56 g of white solid. The solid was recrystallized in water:acetone (1:3 v/v) mixture and dried *in vacuo* at room temperature.

| Analysis calc'd $Ca_3C_{34}H_{58}N_8O_{14}$ (12% $H_2O$): | | | |
|---|---|---|---|
| | C, 39.50; | H, 6.85; | N, 10.84; |
| Found: | C, 39.66; | H, 6.83; | N, 10.76. |

Example 3

Calcium[1,4,7,10-tetraazacyclododecane-1,4,7-10-tetraacetatocalcium(II)], Ca[Ca(DOTA]*

Twenty millimoles of DOTA is dissolved in 40 mL of water and 44 mmoles of $CaCO_3$ is added slowly. The solution is heated under reflux at 80°C for 2 hours and it is cooled to room temperature and filtered to remove excess solid $CaCO_3$. The solution is then rotary evaporated to dryness and placed in a 75°C vacuum oven (5 mm Hg) for 24 hours. A white solid is obtained.

Example 4

Triscalcium bis[N,N'-bis[2-[bis(carboxymethyl)-amino]ethylglycinatecalcium(II)], $Ca_3[Ca(DTPA)]_2$**

Twenty millimoles of DTPA is dissolved in 40 mL of water and 55 mmoles of $CaCO_3$ is added slowly. The solution is heated under reflux at 80°C for 2 hours and it is cooled to room temperature and filtered to remove excess solid $CaCO_3$. The solution is then rotary evaporated to dryness and placed in a 75°C vacuum oven (5 mm Hg) for 24 hours. A white solid is obtained.

Example 5

The relative toxicities of free metal, free ligand, metal chelate contrast agent and excipient of the present invention were determined by injecting samples of each into the tail vein of Charles River CD-1 mice (18-23 g, 25-35 days) at 0.02 mL/g. The observation period was 14 days.
The results are summarized in Table 1.

**HP-DO3A = 1,4,7-tris(carboxymethyl)-10-(2'-hydroxy)propyl-1,4,7,10-tetraazacyclododecanoate.
*DOTA = 1,4,7,10-tetraazacyclododecane-N,N',N",N"'-tetraacetate
**DTPA = N,N-bis[2-[bis(carboxymethyl)-amino]ethylglycinate

Table 1

| Intravenous Acute Tolerance in Mice ($LD_{50}$ Values) for $Gd(OH)_3$, DO3A, DOTA, Gd(DO3A), Gd(DO3A) formulated, Gd(HP-DO3A), Gd(HP-DO3A) formulated, $Ca[Ca(DO3A)]_2$, $Ca[Ca(HP-DO3A)]_2$, and $CaCl_2$ at the Physiologic pH. | |
|---|---|
| Compound | $LD_{50}$ (mMol/kg) |
| $Gd(OH)_3$ | 0.1 |
| DO3A | 0.12 |
| HP-DO3A | 0.11 |
| DOTA | 0.18 |
| Gd(DO3A) | 5.7 |
| Gd(DO3A), formulated[a] | 7.4 (male) |
| | 8.5 (female) |
| Gd(HP-DO3A) | 10 |
| Gd(HP-DO3A), formulated[b] | 10.7 (male) |
| | 13.6 (female) |
| $Ca[Ca(DO3A)]_2$ | 1.6 |
| $Ca[Ca(HP-DO3A)]_2$ | 1.3 |
| $CaCl_2$ | 1.5 |

[a] The formulation consists of Gd(DO3A), 0.5 M; $Ca[Ca(DO3A)]_2$, 0.25 mM; Tris Buffer, 10 mM (pH 7.4).
[b] The formulation consists of Gd(HP-DO3A), 0.5 M; $Ca[Ca(HP-DO3A)]_2$, 0.25 mM; Tris Buffer, 10 mM (pH 7.4).

The data illustrate that the complexed Gd(DO3A) (i.e., metal chelate contrast agent) and calcium complex salt (i.e., excipient) were highly tolerated. Therefore, when using the calcium complex salt of the present invention as the excipient, the amounts of free metal and free ligand are greatly reduced and any excesses of chelated contrast agent or excipient are well tolerated providing contrast methodology with enhanced safety.

Example 6

The following novel diagnostic formulation was prepared by combining the known diagnostic agent, Gadoteridol, with the excipient of Example 2 using the procedure which follows:

| Ingredient | Amount (per ml) |
|---|---|
| Gadoteridol[1] (diagnostic agent) | 558.6 mg |
| Calteridol Calcium[2] (novel excipient) | 0.46 mg |
| Tromethamine (buffer) | 1.21 mg |
| 1N NaOH solution | as needed for pH adjustment |
| 1N HCl solution | as needed for pH adjustment |
| Water for Injection USP q.s. | 1.0 ml |

[1]Gadolinium(III) 1,4,7-tris(carboxymethyl)-10-(2'-hydroxypropyl)-1,4,7,10-tetraazacyclododecane
[2]$Ca[Ca(HP\text{-}DO3A)]_2$ excipient of Example 2 above.

Procedure: About 60% of the necessary water was heated to between 48 and 52°C and vigorously agitated while adding the buffer (tromethamine) and excipient (calteridol calcium). Thereafter, the diagnostic agent (gadoteridol) was added slowly and the agitation was continued for about 1 hour while maintaining the temperature at 48-52°C. The so-prepared solution was cooled to 20-25°C and the pH was adjusted to between 7.3 and 7.5 (7.4 optimum) using HCl solution and/or NaOH solution as necessary. The balance of the water was added and the pH rechecked/readjusted. The solution was filtered through a sterile 0.2 micrometer membrane and thereafter sterilized and stored at controlled room temperature (15-30°C).

Example 7

A novel diagnostic composition for Dotarem, i.e., N-methylglucamine [Gd(DOTA)], was prepared using the procedure above in Example 6 but substituting Dotarem for Gadoteridol and substituting the excipient of Example 3, Ca[Ca(DOTA)], for the calteridol calcium.

Example 8

A novel diagnostic composition for Magnevist, i.e., $(N\text{-methylglucamine})_2$ [Gd(DTPA)], was prepared using the procedure of Example 6 but substituting Magnevist for Gadoteridol and substituting the excipient of Example 4, $Ca_3[Ca(DTPA)]_2$, for the calteridol calcium.

Example 9

A novel diagnostic composition for Gadodiamide, i.e., Gd(DTPA-bis methylamide), was prepared using the procedure of Example 6 but substituting Gadodiamide for Gadoteridol and substituting an excipient $Ca[Ca(DTPA\text{-bis methylamide})]_2$, prepared using the methodology of Example 2.

Example 10

A novel diagnostic composition was prepared for the contrast agent gadolinium DTPA bis morpholinoamide using the procedure of Example 6 substituting this agent for Gadoteridol and substituting an excipient, $Ca[Ca(DTPA\text{ bis morpholinoamide})]_2$ prepared using the methodology of Example 2.

Example 11

A novel diagnostic composition was prepared for the contrast agent gadolinium (DTPA 1,2-dihydroxypropylamide) using the procedure of Example 6 substituting this agent for Gadoteridol and substituting an excipient, $Ca[Ca(DTPA\text{ 1,2-dihydroxypropylamide})]_2$, prepared using the methodology of Example 2.

## Claims

**Claims for the following Contracting States : AT, BE, CH, DE, DK, FR, GB, IT, LI, LU, NL, SE**

1. An excipient for a metal chelate contrast agent, wherein said metal chelate contrast agent, M(L), comprises a metal ion complexed with an organic ligand, which excipient has the formula I

$$X_m[X'(L')]_n \qquad (I)$$

wherein X and X' are each independently selected from calcium or zinc, L' is an organic ligand which may be L or another organic ligand which has a greater affinity for M than for calcium or zinc, and wherein m and n are each independently 1, 2 or 3.

2. The excipient of claim 1 wherein X = X' = calcium .

3. The excipient of claim 1 wherein L and L' are independently selected from linear and macrocyclic polyaminopolycarboxylic acids and derivatives thereof.

4. The excipient of claim 1 wherein L and L' are independently selected from compounds of the formula

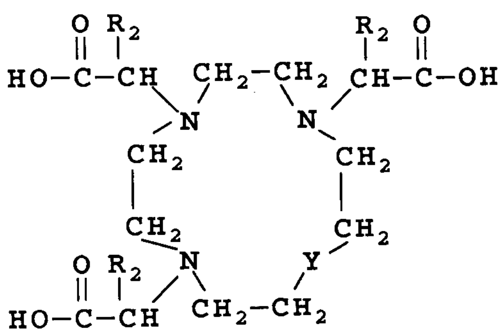

wherein

Y is oxygen or

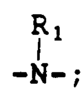

$R_1$ and $R_2$ are each independently hydrogen, alkyl, arylalkyl, aryl, alkoxy, hydroxyalkyl, hydroxyalkoxy,

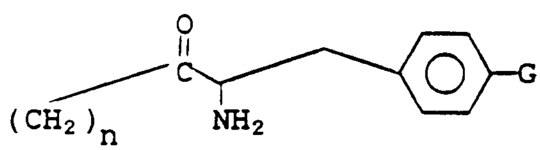 , 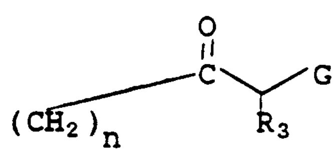

$-(CH_2)_nG$,

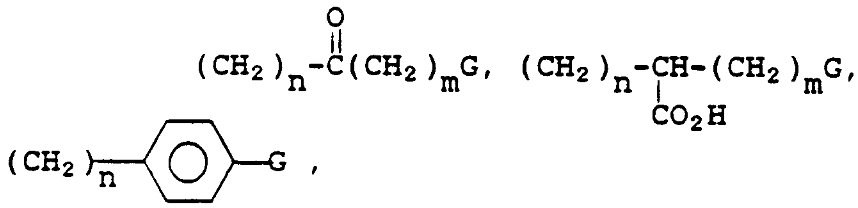

wherein G is $NH_2$, NCS,

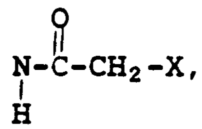

$CO_2H$, $NHR_4$, $N(R_4)_2$, CN, wherein $R_4$ is alkyl or hydroxyalkyl, hydroxyalkoxy,

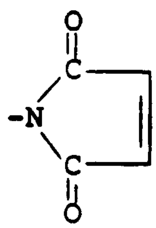 , 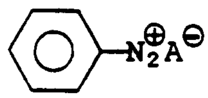

(where A is an anion),

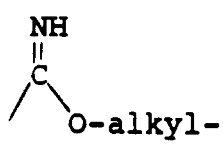 , 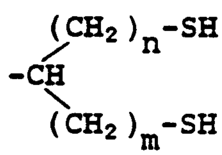 ,

wherein n and m are zero or an integer from one to five, $R_3$ is hydrogen, hydroxyalkyl, alkoxy, alkyl, aryl, arylalkyl or hydroxyalkoxy and X is chloro, bromo or iodo.

5. The excipient of claim 1 wherein L and L' are independently selected from the compounds of the formula

<u>B</u>

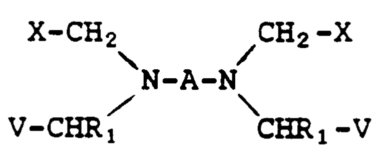

or

<u>C</u>

$N(CH_2X)_3$

wherein

X is -COOY, $PO_3HY$ or -CONHOY;

Y is a hydrogen atom, a metal ion equivalent and/or a physiologically biocompatible cation of an inorganic or organic base or amino acid;

A is $-CHR_2-CHR_3-$, $-CH_2CH_2(ZCH_2-CH_2)_m-$,

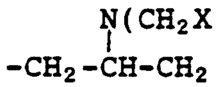 , or 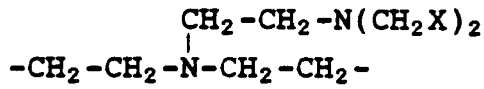 ,

wherein X is as defined above;

each $R_1$ is hydrogen or methyl;

$R_2$ and $R_3$ together represent a trimethylene group or a tetramethylene group or individually are hydrogen atoms, lower alkyl groups (e.g., 1-8 carbons), phenyl groups, benzyl groups or $R_2$ is a hydrogen atom and $R_3$ is $-(CH_2)_p-C_6H_4-W$-protein where p is 0 or 1, W is -NH-, $-NHCOCH_2-$ or -NHCS-, protein represents a protein residue;

m is 1, 2 or 3;

Z is an oxygen atom or a sulfur atom or the group $NCH_2X$ or $NCH_2CH_2OR_4$ wherein X is as defined above and $R_4$ is $C_{1-8}$alkyl;

V is X or is $-CH_2OH$, $-CONH(CH_2)_nX$ or -COB, wherein X is as defined above, B is a protein or lipid residue, n is an integer from 1 to 12, or if $R_1$, $R_2$ and $R_3$ are each hydrogen; then both V's together form the group

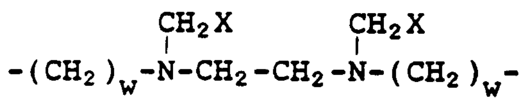

where X is as above, w is 1, 2 or 3, provided that at least two of the substituents Y represent metal ion equivalents of an element with an atomic number of 21 to 29, 42, 44 or 57 to 83.

6. The excipient of claim 1 wherein L and L' are independently selected from the compounds of the formula

D

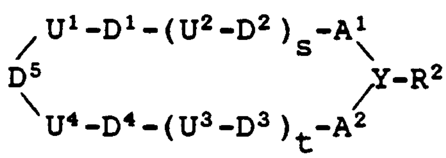

wherein

Y is N or P;

$A^1$ and $A^2$ are each optionally branched $C_{2-6}$ alkylene;

$U^1$, $U^2$, $U^3$ and $U^4$ are each a single bond or optionally branched $C_{1-6}$ alkylene;

$D^1$, $D^2$, $D^3$, $D^4$ are each O, S, $C_{1-6}$ alkylene or $NR_7$;

$R_7$ is hydrogen or $C_{1-4}$ alkylene having a $COOR^1$ terminal group;

$R^1$ is hydrogen or a metal ion equivalent;

$D^5$ is $D^1$ or $CHR^5$, where $R^5$ can be hydrogen or optionally unsaturated $C_{1-20}$ alkylene which may include imino, phenyleneoxy, phenyleneimino, amido, ester, O, S and/or N optionally substituted with OH, SH imino and/or amino and may carry a terminal functional group (optionally bonded to a macromolecule B);

s and t are each 0-5;

$R_2$ is hydrogen, optionally substituted $C_{1-16}$ alkyl, acyl, acylalkyl (optionally substituted by one or more OH or lower alkoxy groups), $-CH_2-X-V$, B or $CH_2COB$ where X is CO, optionally branched $C_{1-10}$ alkylene (optionally substituted by 1 or more OH or lower alkoxy groups) or optionally branched $C_{2-23}$ alkylene interrupted by O;

V is $NR^3R^4$ or $COOR^6$;

$R^3$ and $R^4$ are each hydrogen, $C_{1-16}$ alkyl (optionally substituted by 1 or more OH or lower alkoxy groups) or together complete a 5-6 membered heterocycle optionally containing another heteroatom;

$R_6$ is hydrogen, $C_{1-16}$ saturated, unsaturated, linear branched or cyclic hydrocarbyl, aryl or aralkyl;

$R_2$ or $R_3$ can be bonded by a $C_{2-20}$ alkylene chain (optionally having a terminal carbonyl group, optionally interrupted by 1 or more O or $R^1$ carboxymethylimino, or substituted by one or more OH, lower alkoxy or carboxy lower alkyl groups) to a second macromolecule of the formula

<u>D'</u>

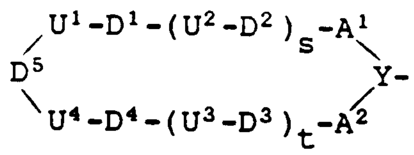

which second macromolecule D' can be the same as or different from the macromolecule of D.

**7.** The excipient of claim 1 wherein L and L' are independently selected from 1,4,7,10-tetraazacyclododecane-1,4,7-triacetic acid, 1,4,7-tris(carboxymethyl)-10-(2'-hydroxypropyl)-1,4,7,10-tetraazacyclododecane, N,N-bis[2-[bis(carboxymethyl)-amino]ethyl]glycine, DTPA-bis methylamide, DTPA bis morpholinoamide and DTPA bis 1,2-dihydroxypropylamide.

**8.** The excipient of claim 1 wherein L and L' are the same organic ligand.

**9.** A contrast agent composition for use in magnetic resonance, x-ray, ultrasound and radiodiagnostic imaging comprising

a metal ion, M, complexed with an organic ligand, L, and a complex salt excipient of the formula

$$X_m[X'(L')]_n$$

in accordance with any one of claims 1 to 8, and a pharmaceutically acceptable carrier there for.

**10.** The composition of claim 9 wherein the mole ratio of said complex salt to said metal chelate contrast agent is between about 0.05 and 10 percent.

**11.** The composition of claim 9 wherein said metal ion is selected from paramagnetic metal atoms, lanthanide series elements, yttrium, and the transition series elements.

**12.** The composition of claim 11 wherein said paramagnetic metals are selected from gadolinium(III), dysprosium(III), manganese(II), manganese(III), chromium(III), iron(II) and iron(III).

**13.** The composition of claim 9 wherein said metal ion complexed with an organic ligand is gadolinium(III) 1,4,7-tris(carboxymethyl)-10-(2'-hydroxypropyl)-1,4,7,10-tetraazacyclododecane and said excipient is calcium bis[1,4,7-tris(carboxymethyl)-10-(2'-hydroxypropyl)-1,4,7,10-tetraazacyclododecanatocalcium(II)];
N-methylglucamine gadolinium (III) 1,4,7,10-tetraazacyclododecane-N,N',N",N'"-tetraacetic acid and said excipient is calcium [1,4,7,10-tetraazacyclododecane-1,4,7,10-tetraacetatocalcium(II)];
di-N-methylglucaminium gadolinium(III) N,N-bis[2-[bis(carboxymethyl)-amino]ethyl]glycine and said excipient is calcium bis[diethylenetriamine-N,N',N',N",N"-pentaacetatocalcium(II)];
diethylene triamine pentaacetato-bis methylamidegadolinium(III) and said excipient is calcium bis[diethylenetriamine-N,N'N',N",N"-pentaacetato-bis methylamide-calcium(II)];
gadolinium(III) 1,4,7,10-tetraazacyclododecane-1,4,7-triacetic acid and said excipient is calcium bis[1,4,7,10-tetraazacyclododecane-1,4,7-triacetatocalcium(II)];
gadolinium (III) DTPA bis morpholinoamide and said excipient is calcium bis [DTPA-bis morpholinamido calcium (II)]; or

gadolinium (III) DTPA bis 1,2-dihydroxypropylamide and said excipient is calcium bis[DTPA bis 1,2-dihydroxypropylamido calcium (II)].

**14.** A contrast agent composition comprising

a metal chelate which is gadolinium (III) 1,4,7-tris(carboxymethyl)-10-(2'-hydroxypropyl)-1,4,7,10-tetraazacyclododecane;
an excipient which is calcium bis[1,4,7-tris(carboxymethyl)-10-(2'-hydroxypropyl)-1,4,7,10-tetraazacyclododecanatocalcium(II)];
a buffer;
acidic and/or basic solution sufficient to adjust pH of said composition to a desired value; and
water.

**Claims for the following Contracting State : ES**

**1.** A method for preparing an excipient for a metal chelate contrast agent, wherein said metal chelate contrast agent, M(L), comprises a metal ion complexed with an organic ligand, which excipient has the formula I

$$X_m[X'(L')]_n \qquad (I)$$

wherein X and X' are each independently selected from calcium or Zinc, L' is an organic ligand which may be L or another organic ligand which has a greater affinity for M than for calcium or zinc, and wherein m and n are each independently 1, 2 or 3, which method comprises reacting the organic ligand L' with a calcium or zinc salt.

**2.** The method of claim 1 for preparing an excipient of the formula I wherein X = X' = calcium .

**3.** The method of claim 1 for preparing an excipient of the formula I wherein L and L' are independently selected from linear and macrocyclic polyaminopolycarboxylic acids and derivatives thereof.

**4.** The method of claim 1 for preparing an excipient of the formula I wherein L and L' are independently selected from compounds of the formula

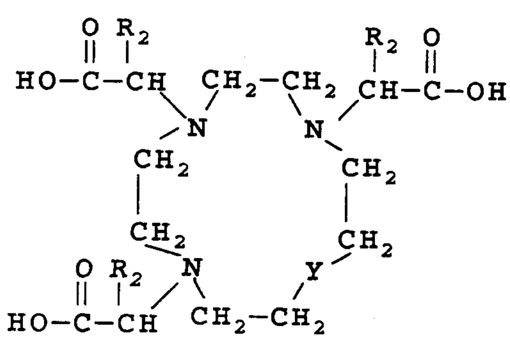

wherein
Y is oxygen or

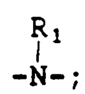

$R_1$ and $R_2$ are each independently hydrogen, alkyl, arylalkyl, aryl, alkoxy, hydroxyalkyl, hydroxyalkoxy,

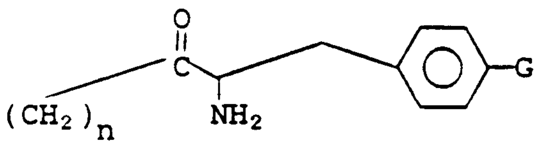, 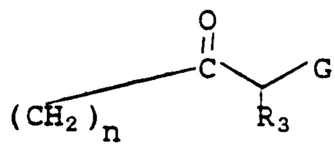

$-(CH_2)_nG$,

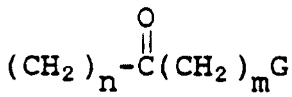, 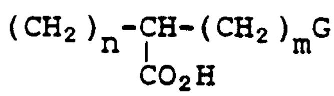,

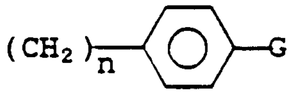,

wherein G is $NH_2$, NCS,

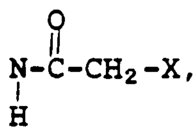,

$CO_2H$, $NHR_4$, $N(R_4)_2$, CN, wherein $R_4$ is alkyl or hydroxyalkyl, hydroxyalkoxy,

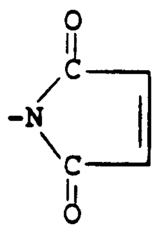, 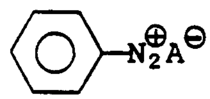

(where A is an anion),

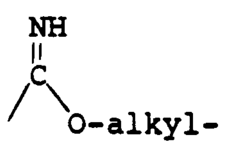, 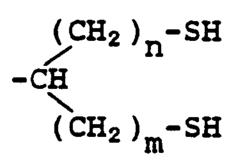,

wherein n and m are zero or an integer from one to five, $R_3$ is hydrogen, hydroxyalkyl, alkoxy, alkyl, aryl, arylalkyl or hydroxyalkoxy and X is chloro, bromo or iodo.

**5.** The method for preparing an excipient of the formula I of claim 1/wherein L and L' are independently selected from the compounds of the formula

B

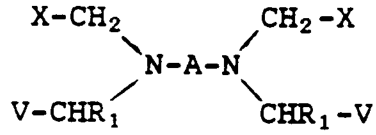

or

C

$$N(CH_2X)_3$$

wherein

X is -COOY, $PO_3HY$ or -CONHOY;

Y is a hydrogen atom, a metal ion equivalent and/or a physiologically biocompatible cation of an inorganic or organic base or amino acid;

A is $-CHR_2-CHR_3-$, $-CH_2CH_2(ZCH_2-CH_2)_m-$,

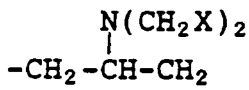 , or 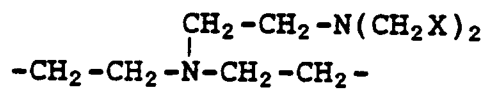 ,

wherein X is as defined above;

each $R_1$ is hydrogen or methyl;

$R_2$ and $R_3$ together represent a trimethylene group or a tetramethylene group or individually are hydrogen atoms, lower alkyl groups (e.g., 1-8 carbons), phenyl groups, benzyl groups or $R_2$ is a hydrogen atom and $R_3$ is $-(CH_2)_p-C_6H_4-W$-protein where p is 0 or 1, W is -NH-, $-NHCOCH_2-$ or -NHCS-, protein represents a protein residue;

m is 1, 2 or 3;

Z is an oxygen atom or a sulfur atom or the group $NCH_2X$ or $NCH_2CH_2OR_4$ wherein X is as defined above and $R_4$ is $C_{1-8}$alkyl;

V is X or is $-CH_2OH$, $-CONH(CH_2)_nX$ or -COB, wherein X is as defined above, B is a protein or lipid residue, n is an integer from 1 to 12, or if $R_1$, $R_2$ and $R_3$ are each hydrogen; then both V's together form the group

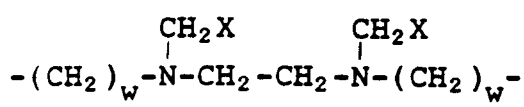

where X is as above, w is 1, 2 or 3, provided that at least two of the substituents Y represent metal ion equivalents of an element with an atomic number of 21 to 29, 42, 44 or 57 to 83.

6. The method of claim 1 for preparing an excipient of the formula I wherein wherein L and L' are independently selected from the compounds of the formula

D

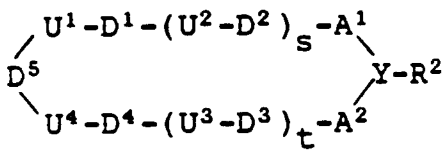

wherein

Y is N or P;

$A^1$ and $A^2$ are each optionally branched $C_{2-6}$ alkylene;

$U^1$, $U^2$, $U^3$ and $U^4$ are each a single bond or optionally branched $C_{1-6}$ alkylene;

$D^1$, $D^2$, $D^3$, $D^4$ are each O, S, $C_{1-6}$ alkylene or $NR_7$;

$R_7$ is hydrogen or $C_{1-4}$ alkylene having a $COOR^1$ terminal group;

$R^1$ is hydrogen or a metal ion equivalent;

$D^5$ is $D^1$ or $CHR^5$, where $R^5$ can be hydrogen or optionally unsaturated $C_{1-20}$ alkylene which may include imino, phenyleneoxy, phenyleneimino, amido, ester, O, S and/or N optionally substituted with OH, SH imino and/or amino and may carry a terminal functional group (optionally bonded to a macromolecule B);

s and t are each 0-5;

$R_2$ is hydrogen, optionally substituted $C_{1-16}$ alkyl, acyl, acylalkyl (optionally substituted by one or more OH or lower alkoxy groups), $-CH_2$-X-V, B or $CH_2COB$ where X is CO, optionally branched $C_{1-10}$ alkylene (optionally substituted by 1 or more OH or lower alkoxy groups) or optionally branched $C_{2-23}$ alkylene interrupted by O;

V is $NR^3R^4$ or $COOR^6$;

$R^3$ and $R^4$ are each hydrogen, $C_{1-16}$ alkyl (optionally substituted by 1 or more OH or lower alkoxy groups) or together complete a 5-6 membered heterocycle optionally containing another heteroatom;

$R_6$ is hydrogen, $C_{1-16}$ saturated, unsaturated, linear branched or cyclic hydrocarbyl, aryl or aralkyl;

$R_2$ or $R_3$ can be bonded by a $C_{2-20}$ alkylene chain (optionally having a terminal carbonyl group, optionally interrupted by 1 or more O or $R^1$ carboxymethylimino, or substituted by one or more OH, lower alkoxy or carboxy lower alkyl groups) to a second macromolecule of the formula

D'

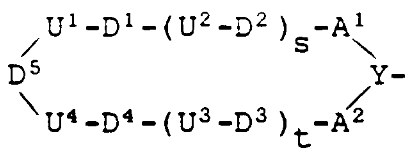

which second macromolecule D' can be the same as or different from the macromolecule of D.

**7.** The method of of claim 1 for preparing an excipient of the formula I wherein L and L' are independently selected from 1,4,7,10-tetraazacyclododecane-1,4,7-triacetic acid, 1,4,7-tris(carboxymethyl)-10-(2'-hydroxypropyl)-1,4,7,10-tetraazacyclododecane, N,N-bis[2-[bis(carboxymethyl)-amino]ethyl]glycine, DTPA-bis methylamide, DTPA bis morpholinoamide and DTPA bis 1,2-dihydroxypropylamide.

**8.** The method of claim 1 for preparing an excipient of the formula I wherein L and L' are the same organic ligand.

**9.** A method for preparing a contrast agent composition for use in magnetic resonance, x-ray, ultrasound and radiodiagnostic imaging comprising combining

a metal ion, M, complexed with an organic ligand, L, and a complex salt excipient of the formula

$$X_m[X'(L')]_n$$

in accordance with any one of claims 1 to 8 with a pharmaceutically acceptable carrier therefor.

10. The method of claim 9 wherein the mole ratio of said complex salt to said metal chelate contrast agent is between about 0.05 and 10 percent.

11. The method of claim 9 wherein said metal ion is selected from paramagnetic metal atoms, lanthanide series elements, yttrium, and the transition series elements.

12. The method of claim 11 wherein said paramagnetic metals are selected from gadolinium(III), dysprosium(III), manganese(II), manganese(III), chromium(III), iron(II) and iron(III).

13. The method of claim 9 wherein said metal ion complexed with an organic ligand is gadolinium(III) 1,4,7-tris(carboxymethyl)-10-(2'-hydroxypropyl)-1,4,7,10-tetraazacyclododecane and said excipient is calcium bis[1,4,7-tris(carboxymethyl )-10-(2'-hydroxypropyl)-1,4,7,10-tetraazacyclododecanatocalcium(II)];
N-methylglucamine gadolinium (III) 1,4,7,10-tetraazacyclododecane-N,N',N",N'"-tetraacetic acid and said excipient is calcium [1,4,7,10-tetraazacyclododecane-1,4,7,10-tetraacetatocalcium(II)];
di-N-methylglucaminium gadolinium(III) N,N-bis[2-[bis(carboxymethyl)-amino]ethyl]glycine and said excipient is calcium bis[diethylenetriamine-N,N',N',N",N"-pentaacetatocalcium(II)];
diethylene triamine pentaacetato-bis methylamidegadolinium(III) and said excipient is calcium bis[diethylenetriamine-N,N'N',N",N"-pentaacetato-bis methylamide-calcium(II)];
gadolinium(III) 1,4,7,10-tetraazacyclododecane-1,4,7-triacetic acid and said excipient is calcium bis[1,4,7,10-tetraazacyclododecane-1,4,7-triacetatocalcium(II)];
gadolinium (III) DTPA bis morpholinoamide and said excipient is calcium bis [DTPA-bis morpholinamido calcium (II)]; or
gadolinium (III) DTPA bis 1,2-dihydroxypropylamide and said excipient is calcium bis[DTPA bis 1,2-dihydroxypropylamido calcium (II)].

14. A method for preparing a contrast agent composition comprising combining a metal chelate which is gadolinium (III) 1,4,7-tris(carboxymethyl)-10-(2'-hydroxypropyl)-1,4,7,10-tetraazacyclododecane;
an excipient which is calcium bis[1,4,7-tris(carboxymethyl)-10-(2'-hydroxypropyl)-1,4,7,10-tetraazacyclododecanatocalcium(II)];
a buffer;
acidic and/or basic solution sufficient to adjust pH of said composition to a desired value; and
water.

**Claims for the following Contracting State : GR**

1. An excipient for a metal chelate contrast agent, wherein said metal chelate contrast agent, M(L), comprises a metal ion complexed with an organic ligand, which excipient has the formula I

$$X_m[X'(L')]_n \qquad (I)$$

wherein X and X' are each independently selected from calcium or zinc, L' is an organic ligand which may be L or another organic ligand which has a greater affinity for M than for calcium or zinc, and wherein m and n are each independently 1, 2 or 3.

2. The excipient of claim 1 wherein X = X' = calcium .

3. The excipient of claim 1 wherein L and L' are independently selected from linear and macrocyclic polyaminopolycarboxylic acids and derivatives thereof.

**4.** The excipient of claim 1 wherein L and L' are independently selected from compounds of the formula

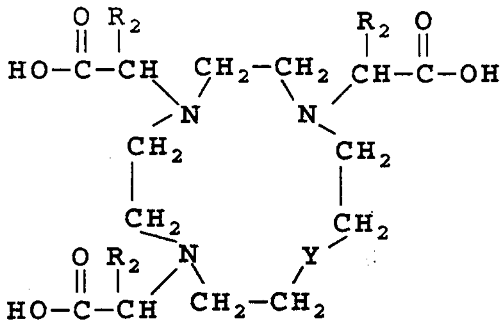

wherein

Y is oxygen or

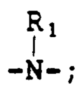

$R_1$ and $R_2$ are each independently hydrogen, alkyl, arylalkyl, aryl, alkoxy, hydroxyalkyl, hydroxyalkoxy,

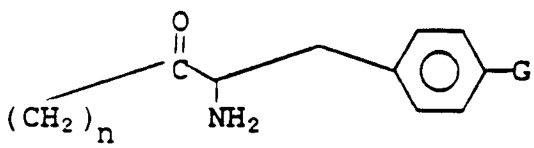

,

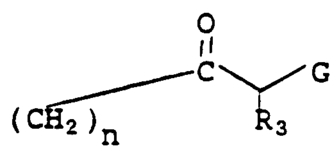

$-(CH_2)_nG$,

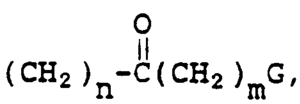

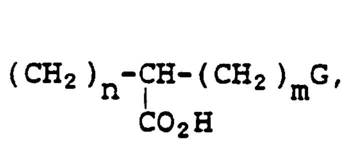

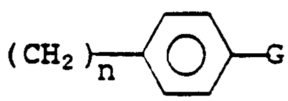

,

wherein G is $NH_2$, NCS,

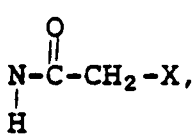

$CO_2H$, $NHR_4$, $N(R_4)_2$, CN, wherein $R_4$ is alkyl or hydroxyalkyl, hydroxyalkoxy,

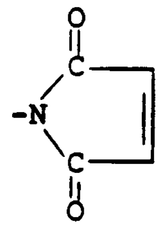 , 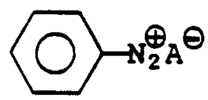

(where A is an anion),

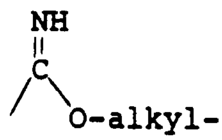 , 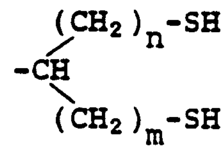 ,

wherein n and m are zero or an integer from one to five, $R_3$ is hydrogen, hydroxyalkyl, alkoxy, alkyl, aryl, arylalkyl or hydroxyalkoxy and X is chloro, bromo or iodo.

**5.** The excipient of claim 1 wherein L and L' are independently selected from the compounds of the formula

B

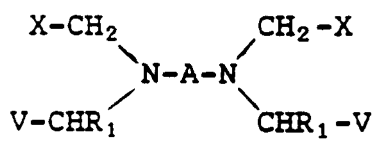

or

C

$$N(CH_2X)_3$$

wherein

X is -COOY, $PO_3HY$ or -CONHOY;

Y is a hydrogen atom, a metal ion equivalent and/or a physiologically biocompatible cation of an inorganic or organic base or amino acid;

A is $-CHR_2-CHR_3-$, $-CH_2CH_2(ZCH_2-CH_2)_m-$,

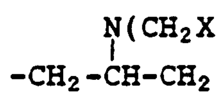 , or 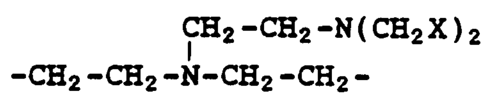 ,

wherein X is as defined above;

each $R_1$ is hydrogen or methyl;

$R_2$ and $R_3$ together represent a trimethylene group or a tetramethylene group or individually are hydrogen atoms, lower alkyl groups (e.g., 1-8 carbons), phenyl groups, benzyl groups or $R_2$ is a hydrogen atom and $R_3$ is $-(CH_2)_p-C_6H_4-W-$protein where p is 0 or 1, W is -NH-, $-NHCOCH_2-$ or -NHCS-, protein represents a protein residue;

m is 1, 2 or 3;

Z is an oxygen atom or a sulfur atom or the group $NCH_2X$ or $NCH_2CH_2OR_4$ wherein X is as defined above and $R_4$ is $C_{1-8}$alkyl;

V is X or is $-CH_2OH$, $-CONH(CH_2)_nX$ or -COB, wherein X is as defined above, B is a protein or lipid residue, n is an integer from 1 to 12, or if $R_1$, $R_2$ and $R_3$ are each hydrogen; then both V's together form the group

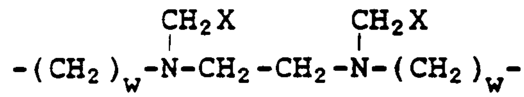

where X is as above, w is 1, 2 or 3, provided that at least two of the substituents Y represent metal ion equivalents of an element with an atomic number of 21 to 29, 42, 44 or 57 to 83.

**6.** The excipient of claim 1 wherein L and L' are independently selected from the compounds of the formula

D

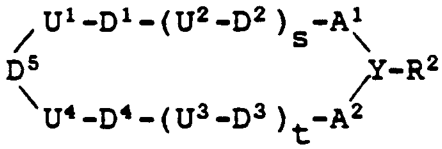

wherein

Y is N or P;

$A^1$ and $A^2$ are each optionally branched $C_{2-6}$ alkylene;

$U^1$, $U^2$, $U^3$ and $U^4$ are each a single bond or optionally branched $C_{1-6}$ alkylene;

$D^1$, $D^2$, $D^3$, $D^4$ are each O, S, $C_{1-6}$ alkylene or $NR_7$;

$R_7$ is hydrogen or $C_{1-4}$ alkylene having a $COOR^1$ terminal group;

$R^1$ is hydrogen or a metal ion equivalent;

$D^5$ is $D^1$ or $CHR^5$, where $R^5$ can be hydrogen or optionally unsaturated $C_{1-20}$ alkylene which may include imino, phenyleneoxy, phenyleneimino, amido, ester, O, S and/or N optionally substituted with OH, SH imino and/or amino and may carry a terminal functional group (optionally bonded to a macromolecule B);

s and t are each 0-5;

$R_2$ is hydrogen, optionally substituted $C_{1-16}$ alkyl, acyl, acylalkyl (optionally substituted by one or more OH or lower alkoxy groups), $-CH_2$-X-V, B or $CH_2COB$ where X is CO, optionally branched $C_{1-10}$ alkylene (optionally substituted by 1 or more OH or lower alkoxy groups) or optionally branched $C_{2-23}$ alkylene interrupted by O;

V is $NR^3R^4$ or $COOR^6$;

$R^3$ and $R^4$ are each hydrogen, $C_{1-16}$ alkyl (optionally substituted by 1 or more OH or lower alkoxy groups) or together complete a 5-6 membered heterocycle optionally containing another heteroatom;

$R_6$ is hydrogen, $C_{1-16}$ saturated, unsaturated, linear branched or cyclic hydrocarbyl, aryl or aralkyl;

$R_2$ or $R_3$ can be bonded by a $C_{2-20}$ alkylene chain (optionally having a terminal carbonyl group, optionally interrupted by 1 or more O or $R^1$ carboxymethylimino, or substituted by one or more OH, lower alkoxy or carboxy lower alkyl groups) to a second macromolecule of the formula

D'

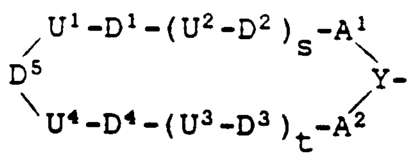

which second macromolecule D' can be the same as or different from the macromolecule of D.

7. The excipient of claim 1 wherein L and L' are independently selected from 1,4,7,10-tetraazacyclododecane-1,4,7-triacetic acid, 1,4,7-tris(carboxymethyl)-10-(2'-hydroxypropyl)-1,4,7,10-tetraazacyclododecane, N,N-bis[2-[bis(carboxymethyl)-amino]ethyl]glycine, DTPA-bis methylamide, DTPA bis morpholinoamide and DTPA bis 1,2-dihydroxypropylamide.

8. The excipient of claim 1 wherein L and L' are the same organic ligand.

9. A method for preparing a contrast agent composition for use in magnetic resonance, x-ray, ultrasound and radiodiagnostic imaging comprising combining
a metal ion, M, complexed with an organic ligand, L, and a complex salt excipient of the formula

$$X_m[X'(L')]_n$$

in accordance with any one of claims 1 to 8 with a pharmaceutically acceptable carrier therefor.

10. The method of claim 9 wherein the mole ratio of said complex salt to said metal chelate contrast agent is between about 0.05 and 10 percent.

11. The method of claim 9 wherein said metal ion is selected from paramagnetic metal atoms, lanthanide series elements, yttrium, and the transition series elements.

12. The method of claim 11 wherein said paramagnetic metals are selected from gadolinium(III), dysprosium(III), manganese(II), manganese(III), chromium(III), iron(II) and iron(III).

13. The method of claim 9 wherein said metal ion complexed with an organic ligand is gadolinium(III) 1,4,7-tris(carboxymethyl)-10-(2'-hydroxypropyl)-1,4,7,10-tetraazacyclododecane and said excipient is calcium bis[1,4,7-tris(carboxymethyl)-10-(2'-hydroxypropyl)-1,4,7,10-tetraazacyclododecanatocalcium(II)];
N-methylglucamine gadolinium (III) 1,4,7,10-tetraazacyclododecane-N,N',N'',N'''-tetraacetic acid and said excipient is calcium [1,4,7,10-tetraazacyclododecane-1,4,7,10-tetraacetatocalcium(II)];
di-N-methylglucaminium gadolinium(III) N,N-bis[2-[bis(carboxymethyl)-amino]ethyl]glycine and said excipient is calcium bis[diethylenetriamine-N,N',N',N'',N''-pentaacetatocalcium(II)];
diethylene triamine pentaacetato-bis methylamide-gadolinium(III) and said excipient is calcium bis[diethylenetriamine-N,N'N',N'',N''-pentaacetato-bis methylamide-calcium(II)];
gadolinium(III) 1,4,7,10-tetraazacyclododecane-1,4,7-triacetic acid and said excipient is calcium bis[1,4,7,10-tetraazacyclododecane-1,4,7-triacetatocalcium(II)];
gadolinium (III) DTPA bis morpholinoamide and said excipient is calcium bis [DTPA-bis morpholinamido calcium (II)]; or
gadolinium (III) DTPA bis 1,2-dihydroxypropylamide and said excipient is calcium bis[DTPA bis 1,2-dihydroxypropylamido calcium (II)].

14. A method for preparing a contrast agent composition comprising combining a metal chelate which is gadolinium (III) 1,4,7-tris(carboxymethyl)-10-(2'-hydroxypropyl)-1,4,7,10-tetraazacyclododecane;
an excipient which is calcium bis[1,4,7-tris(carboxymethyl)-10-(2'-hydroxypropyl)-1,4,7,10-tetraazacyclododecanatocalcium(II)];
a buffer;
acidic and/or basic solution sufficient to adjust pH of said composition to a desired value; and
water.

**Patentansprüche**

**Patentansprüche für folgende Vertragsstaaten : AT, BE, CH, DE, DK, FR, GB, IT, LI, LU, NL, SE**

1. Zusatzstoff für ein Metallchelat-Kontrastmittel, bei dem das Metallchelat-Kontrastmittel M(L) ein Metallion umfaßt, das mit einem organischen Ligand komplexiert ist, und der Zusatzstoff die Formel I

$$X_m[X'(L')]_n \qquad (I)$$

aufweist, in der X und X' jeweils unabhängig voneinander aus Calcium oder Zink ausgewählt sind, L' ein organischer Ligand ist, der L oder ein anderer organischer Ligand mit einer größeren Affinität zu M als zu Calcium oder Zink sein kann, und in der m und n jeweils unabhängig voneinander 1, 2 oder 3, sind.

**2.** Zusatzstoff nach Anspruch 1, bei dem X = X' = Calcium ist.

**3.** Zusatzstoff nach Anspruch 1, bei dem L und L' jeweils unabhängig voneinander aus linearen und makrocyclischen Polyaminopolycarbonsäuren und deren Derivaten ausgewählt sind.

**4.** Zusatzstoff nach Anspruch 1, bei dem L und L' jeweils unabhängig voneinander aus Verbindungen der Formel

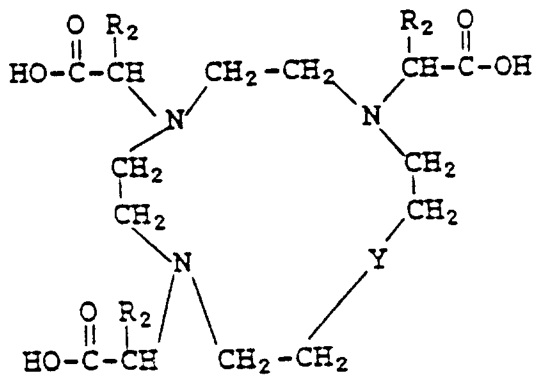

ausgewählt sind, in der

Y Sauerstoff oder

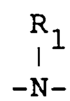

ist,

$R_1$ und $R_2$ jeweils unabhängig Wasserstoff, Alkyl, Arylalkyl, Aryl, Alkoxy, Hydroxyalkyl, Hydroxyalkoxy,

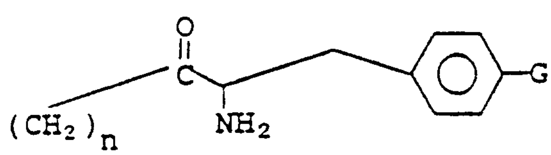

,

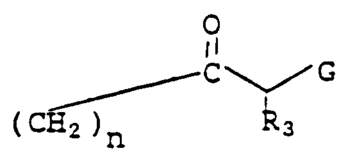

$-(CH_2)_nG$,

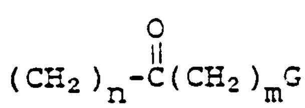

,

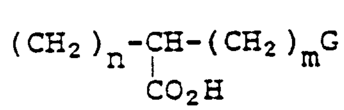

,

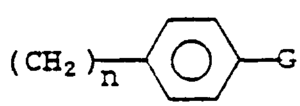

,

sind, in denen G $NH_2$, NCS,

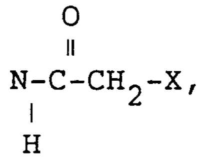

$CO_2H$, $NHR_4$, $N(R_4)_2$, CN ist, wobei $R_4$ Alkyl oder Hydroxyalkyl, Hydroxyalkoxy,

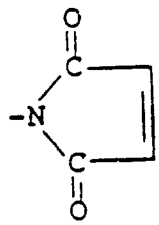 , 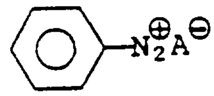

(in der A ein Anion ist),

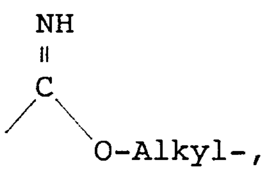 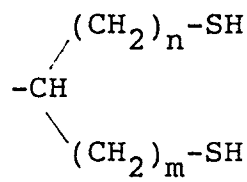

ist, wobei n und m 0 oder eine ganze Zahl von 1 bis 5, $R_3$ Wasserstoff, Hydroxyalkyl, Alkoxy, Alkyl, Aryl, Arylalkyl oder Hydroxyalkoxy und X Chlor, Brom oder Iod ist.

**5.** Zusatzstoff nach Anspruch 1, bei dem L und L' jeweils unabhängig voneinander ausgewählt sind aus Verbindungen der Formel

B

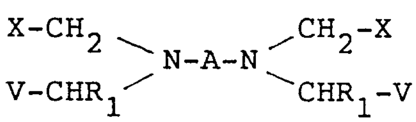

oder

C

$$N(CH_2X)_3,$$

in denen

X -COOY, $PO_3HY$ oder -CONHOY ist,

Y ein Wasserstoffatom, ein Metallion-Äquivalent und/oder ein physiologisch biokompatibles Kation einer anorganischen oder organischen Base oder Aminosäure ist,

A $-CHR_2-CHR_3-$, $-CH_2CH_2(ZCH_2-CH_2)_m-$,

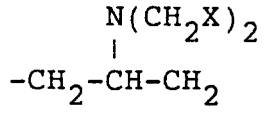

oder

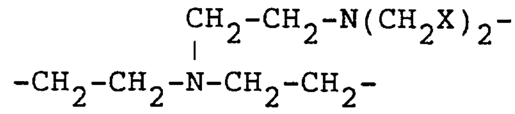

ist, wobei

X wie oben definiert ist,

jedes $R_1$ Wasserstoff oder Methyl ist,

$R_2$ und $R_3$ zusammen eine Trimethylengruppe oder eine Tetramethylengruppe darstellen oder unabhängig voneinander Wasserstoffatome, niedere Alkylgruppen (z.B. 1 bis 8-Kohlenwasserstoffe), Phenylgruppen, Benzylgruppen oder $R_2$ ein Wasserstoffatom ist und $R_3$ $-(CH_2)_p-C_6H_4-W$-Protein ist, wobei p 0 oder 1, W -NH-, $-NHCOCH_2-$ oder -NHCS- ist, Protein ein Proteinrest darstellt,

m 1, 2 oder 3 ist,

Z ein Sauerstoffatom oder ein Schwefelatom oder die Gruppe $NCH_2X$ oder $NCH_2CH_2OR_4$ ist, wobei X wie oben definiert ist und $R_4$ ein $C_1$- bis $C_8$-Alkyl ist,

V X oder $-CH_2OH$, $-CONH(CH_2)_nX$ oder -COB ist, wobei X wie oben definiert ist, B ein Protein- oder Lipidrest ist, n eine ganze Zahl von 1 bis 12 ist, oder, wenn $R_1$, $R_2$ und $R_3$ jeweils Wasserstoff sind, beide V's zusammen die Gruppe

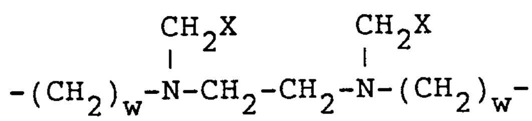

bilden,

in der X wie oben ist, w 1, 2 oder 3 ist, mit der Maßgabe, daß mindestens zwei der Substituenten Y Metallionen-Äquivalente eines Elements mit einer Ordnungszahl von 21 bis 29, 42, 44 oder 57 bis 83 darstellen.

6. Zusatzstoff nach Anspruch 1, bei dem L und L' jeweils unabhängig voneinander ausgewählt sind aus Verbindungen der Formel

<u>D</u>

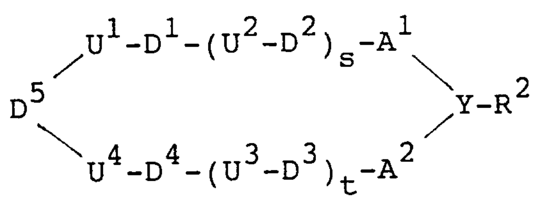

,

in der

Y N oder P ist,

$A^1$ und $A^2$ jeweils gegebenenfalls verzweigtes $C_2$- bis $C_6$-Alkylen sind,

$U^1$, $U^2$, $U^3$ und $U^4$ jeweils einfach gebundenes oder gegebenenfalls verzweigtes $C_1$- bis $C_6$-Alkylen sind,

$D^1$, $D^2$, $D^3$, $D^4$ jeweils O, S, $C_1$- bis $C_6$-Alkylen oder $NR_7$ sind,

$R_7$ Wasserstoff oder $C_1$- bis $C_4$-Alkylen mit einer $COOR^1$-Endgruppe ist,

$R^1$ Wasserstoff oder ein Metallion-Äquivalent ist,

$^{D5}$ $D^1$ oder $CHR^5$ ist, wobei $R^5$ Wasserstoff oder gegebenenfalls ungesättigtes $C_1$- bis $C_{20}$-Alkylen sein kann, das Imino, Phenylenoxy, Phenylenimino, Amido, Ester, O, S und/oder N, gegebenenfalls substituiert mit OH, SH, Imino und/oder Amino ist, und eine funktionelle Endgruppe tragen kann (die gegebenenfalls an ein Makromolekül B gebunden ist),

s und t jeweils 0 bis 5 sind,

$R_2$ Wasserstoff, gegebenenfalls substituiertes $C_1$- bis $C_{16}$-Alkyl, Acyl, Acylalkyl (gegebenenfalls substituiert mit einer oder mehreren OH- oder niederen Alkoxygruppen), -$CH_2$-X-V, B oder $CH_2COB$ ist, wobei X CO, gegebenenfalls verzweigtes $C_1$- bis $C_{10}$-Alkylen (gegebenenfalls substituiert mit einer oder mehreren OH- oder niederen Alkoxygruppen) oder gegebenenfalls verzweigtes $C_2$- bis $C_{23}$-Alkylen, unterbrochen durch O, ist,

V $NR^3R^4$ oder $COOR^6$ ist,

$R^3$ und $R^4$ jeweils Wasserstoff, $C_1$- bis $C_{16}$-Alkyl (gegebenenfalls substituiert mit einer oder mehreren OH- oder niederen Alkylgruppen) sind oder zusammen einen 5- bis 6-gliedrigen Heterozyklus bilden, der gegebenenfalls ein weiteres Heteroatom enthält,

$R^6$ Wasserstoff, $C_1$- bis $C_{16}$-, gesättigte, ungesättigte, lineare, verzweigte oder cyclische Kohlenwasserstoff-, Aryl- oder Aralkyl-Gruppe ist,

$R_2$ oder $R_3$ durch eine $C_2$- bis $C_{20}$-Alkylenkette (die gegebenenfalls eine terminale Carbonylgruppe enthält, welche gegebenenfalls durch eine oder mehrere O- oder $R^1$-Carboxymethylimino unterbrochen sind oder mit einer oder mehreren OH-, niederen Alkoxy- oder Carboxy-niederen Alkyl-Gruppen substituiert sind) an ein zweites Makromolekül der Formel

D'

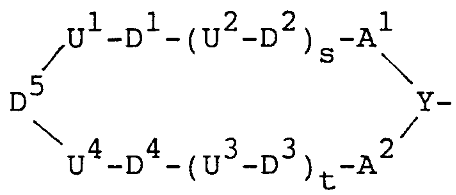

gebunden ist, welches gleich dem Makromolekül D oder von ihm verschieden sein kann.

7. Zusatzstoff nach Anspruch 1, bei dem L und L' jeweils unabhängig voneinander ausgewählt sind aus 1,4,7,10-Tetraazacyclododecan-1,4,7-triessigsäure, 1,4,7-Tris-(carboxymethyl)-10-(2'-hydroxypropyl)-1,4,7,10-tetraazacyclododecan, N,N-Bis[2-[bis(carboxymethyl)-amino]ethyl]glycin, DTPA-bis-methylamid, DTPA-bis-morpholinoamid und DTPA-bis-1,2-dihydroxypropylamid.

8. Zusatzstoff nach Anspruch 1, bei dem L und L' der gleiche organische Ligand sind.

9. Kontrastmittelzusammensetzung zur Verwendung bei der bildgebenden magnetischen Resonanz-, Röntgen-, Ultraschall- und Radiodiagnostik-Untersuchung, umfassend

ein Metallion M, das mit einem organischen Ligand L komplexiert ist, und einen Komplexsalz-Zusatzstoff der Formel

$$X_m[X'(L')]_n$$

gemäß einem der Ansprüche 1 bis 8 sowie

einen pharmazeutisch akzeptablen Träger dafür.

10. Zusammensetzung nach Anspruch 9, bei dem das Molverhältnis des Komplexsalzes zu Metallchelat-Kontrastmittel zwischen etwa 0,05 und 10% liegt.

11. Zusammensetzung nach Anspruch 9, bei dem das Metallion ausgewählt ist aus paramagnetischen Metallatomen, Elementen der Lanthanidenreihe, Yttrium und den Elementen der Übergangsreihe.

12. Zusammensetzung nach Anspruch 11, bei dem die paramagnetischen Metalle ausgewählt sind aus Gadolinium(III), Dysprosium(III), Mangan(II), Mangan(III), Chrom(III), Eisen(II) und Eisen(III).

13. Zusammensetzung nach Anspruch 9, bei dem das mit einem organischen Liganden komplexierte Metallion Gadolinium(III)-1,4,7-tris(carboxymethyl)-10-(2'-hydroxypropyl)-1,4,7,10-tetraazacyclododecan und der Zusatzstoff Calcium-bis[1,4,7-tris(carboxymethyl)-10-(2'-hydroxypropyl)-1,4,7,10-tetraazacyclododecanatocalcium(II)] ist, N-Methylglucamin-gadolinium(III)-1,4,7,10-tetraazacyclododecan-N,N',N'',N'''-tetraessigsäure und der Zusatzstoff

Calcium-[1,4,7,10-tetraazacyclododecan-1,4,7,19-tetraacetatocalcium(II)] ist, Di-N-methylglucaminium-gadolinium(III)-N,N-bis[2-[bis(carboxymethyl)-amino]ethyl]glycin und der Zusatzstoff Calcium-bis[diethylentriamin-N,N',N',N'',N''-pentacetatocalcium(II)] ist, Diethylentriaminpentaacetato-bis-methylamid-gadolinium(III) und der Zusatzstoff Calcium-bis[diethylentriamin-N,N',N',N'',N''-pentaacetato-bis-methylamid-calcium(II)] ist, Gadolinium(III)-1,4,7,10-tetraazacyclododecan-1,4,7-triessigsäure und der Zusatzstoff Calcium-bis[1,4,7,10-tetraazacyclododecan-1,4,7-triacetatocalcium(II)] ist, Gadolinium(III)-DTPA-bis-morpholinoamid und der Zusatzstoff Calcium-bis-[DTPA-bis-morpholinamido-calcium(II)] ist oder Gadolinium(III)-DTPA-bis-1,2-dihydroxypropylamid und der Zusatzstoff Calcium-bis[DTPA-bis-1,2-dihydroxypropylamidocalcium(II)] ist.

**14.** Kontrastmittelzusammensetzung umfassend
ein Metallchelat, das Gadolinium(III)-1,4,7-tris(carboxymethyl)-10-(2'-hydroxypropyl)-1,4,7,10-tetraazacyclododecan ist,
einen Zusatzstoff, der Calcium-bis[1,4,7-tris(carboxymethyl)-10-(2'-hydroxypropyl)-1,4,7,10-tetraazacyclododecanatocalcium(II)] ist,
einen Puffer,
ausreichend saure und/oder basische Lösung, um den pH-Wert der Zusammensetzung auf einen gewünschten Wert einzustellen und
Wasser.

**Patentansprüche für folgenden Vertragsstaat : ES**

**1.** Verfahren zur Herstellung eines Zusatzstoffes für ein Metallchelat-Kontrastmittel, wobei das Metallchelat-Kontrastmittel M(L) ein Metallion umfaßt, das mit einem organischen Ligand komplexiert ist, und der Zusatzstoff die Formel I

$$X_m[X'(L')]_n \qquad (I)$$

aufweist, in der X und X' jeweils unabhängig voneinander aus Calcium oder Zink ausgewählt sind, L' ein organischer Ligand ist, der L oder ein anderer organischer Ligand mit einer größeren Affinität zu M als zu Calcium oder Zink sein kann, und in der m und n jeweils unabhängig voneinander 1, 2 oder 3, sind, bei dem der organische Ligand L' mit Calcium- oder Zinksalz umgesetzt wird.

**2.** Verfahren nach Anspruch 1 zur Herstellung eines Zusatzstoffes der Formel I, bei dem X = X' = Calcium ist.

**3.** Verfahren nach Anspruch 1 zur Herstellung eines Zusatzstoffes der Formel I, bei dem L und L' jeweils unabhängig voneinander aus linearen und makrocyclischen Polyaminopolycarbonsäuren und deren Derivaten ausgewählt sind.

**4.** Verfahren nach Anspruch 1 zur Herstellung eines Zusatzstoffes der Formel I, bei dem L und L' jeweils unabhängig voneinander aus Verbindungen der Formel

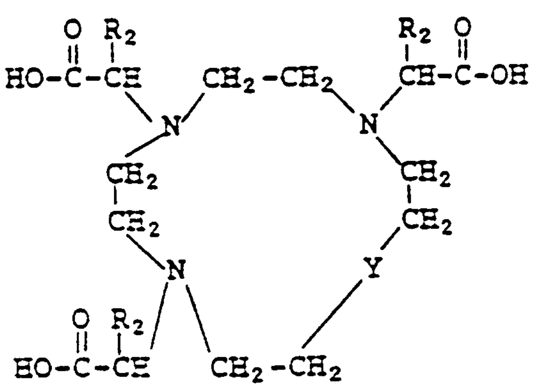

ausgewählt sind, in der

Y Sauerstoff oder

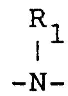

ist,

$R_1$ und $R_2$ jeweils unabhängig Wasserstoff, Alkyl, Arylalkyl, Aryl, Alkoxy, Hydroxyalkyl, Hydroxyalkoxy,

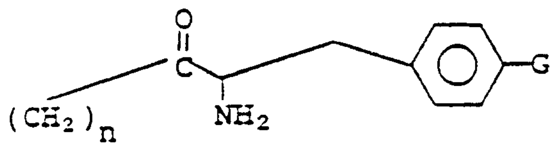 , 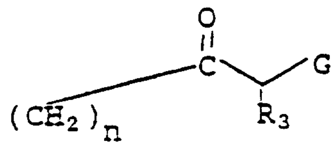

$-(CH_2)_nG$,

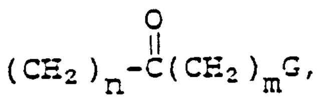 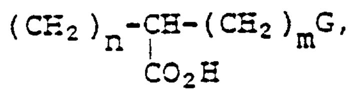

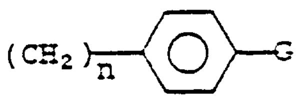 ,

sind, in denen G $NH_2$, NCS,

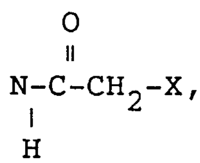

$CO_2H$, $NHR_4$, $N(R_4)_2$, CN ist, wobei $R_4$ Alkyl oder Hydroxyalkyl, Hydroxyalkoxy,

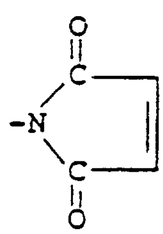 , 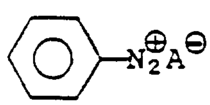

(in der A ein Anion ist),

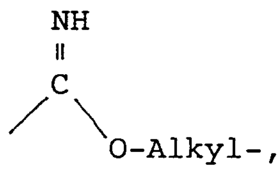

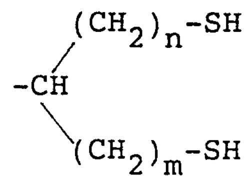

ist, wobei n und m 0 oder eine ganze Zahl von 1 bis 5, $R_3$ Wasserstoff, Hydroxyalkyl, Alkoxy, Alkyl, Aryl, Arylalkyl oder Hydroxyalkoxy und X Chlor, Brom oder Iod ist.

**5.** Verfahren nach Anspruch 1 zur Herstellung eines Zusatzstoffes der Formel I, bei dem L und L' jeweils unabhängig voneinander ausgewählt sind aus Verbindungen der Formel

B

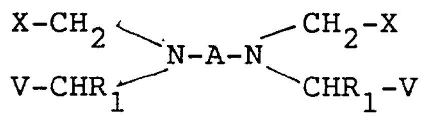

oder

C

$N(CH_2X)_3$,

in denen

X -COOY, $PO_3HY$ oder -CONHOY ist,

Y ein Wasserstoffatom, ein Metallion-Äquivalent und/oder ein physiologisch biokompatibles Kation einer anorganischen oder organischen Base oder Aminosäure ist,

A $-CHR_2-CHR_3-$, $-CH_2CH_2(ZCH_2-CH_2)_m-$,

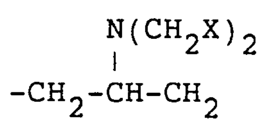

oder

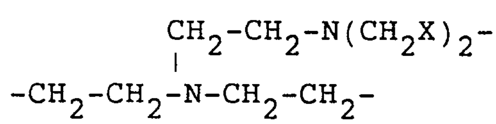

ist, wobei

X wie oben definiert ist,

jedes $R_1$ Wasserstoff oder Methyl ist,

$R_2$ und $R_3$ zusammen eine Trimethylengruppe oder eine Tetramethylengruppe darstellen oder unabhängig voneinander Wasserstoffatome, niedere Alkylgruppen (z.B. 1 bis 8-Kohlenwasserstoffe), Phenylgruppen, Benzylgruppen oder $R_2$ ein Wasserstoffatom ist und $R_3$ $-(CH_2)_p-C_6H_4-W-$Protein ist, wobei p 0 oder 1, W -NH-, $-NHCOCH_2-$ oder -NHCS- ist, Protein ein Proteinrest darstellt,

m 1, 2 oder 3 ist,

Z ein Sauerstoffatom oder ein Schwefelatom oder die Gruppe $NCH_2X$ oder $NCH_2CH_2OR_4$ ist, wobei X wie oben definiert ist und $R_4$ ein $C_1$- bis $C_8$-Alkyl ist,

V X oder $-CH_2OH$, $-CONH(CH_2)_nX$ oder -COB ist, wobei X wie oben definiert ist, B ein Protein- oder Lipidrest ist, n eine ganze Zahl von 1 bis 12 ist, oder, wenn $R_1$, $R_2$ und $R_3$ jeweils Wasserstoff sind, beide V's zusammen die Gruppe

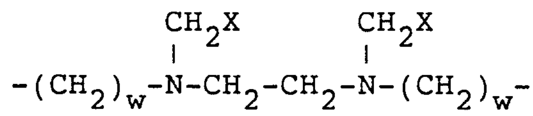

bilden,
in der X wie oben ist, w 1, 2 oder 3 ist, mit der Maßgabe, daß mindestens zwei der Substituenten Y Metallionen-Äquivalente eines Elements mit einer Ordnungszahl von 21 bis 29, 42, 44 oder 57 bis 83 darstellen.

6. Verfahren nach Anspruch 1 zur Herstellung eines Zusatzstoffes der Formel I, bei dem L und L' jeweils unabhängig voneinander ausgewählt sind aus Verbindungen der Formel

D

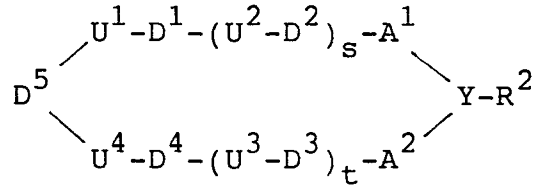

,

in der

Y N oder P ist,

$A^1$ und $A^2$ jeweils gegebenenfalls verzweigtes $C_2$- bis $C_6$-Alkylen sind,

$U^1$, $U^2$, $U^3$ und $U^4$ jeweils einfach gebundenes oder gegebenenfalls verzweigtes $C_1$- bis $C_6$-Alkylen sind,

$D^1$, $D^2$, $D^3$, $D^4$ jeweils O, S, $C_1$- bis $C_6$-Alkylen oder $NR_7$ sind,

$R_7$ Wasserstoff oder $C_1$- bis $C_4$-Alkylen mit einer $COOR^1$-Endgruppe ist,

$R^1$ Wasserstoff oder ein Metallion-Äquivalent ist,

$D^5$ $D^1$ oder $CHR^5$ ist, wobei $R^5$ Wasserstoff oder gegebenenfalls ungesättigtes $C_1$- bis $C_{20}$-Alkylen sein kann, das Imino, Phenylenoxy, Phenylenimino, Amido, Ester, O, S und/oder N, gegebenenfalls substituiert mit OH, SH, Imino und/oder Amino ist, und eine funktionelle Endgruppe tragen kann (die gegebenenfalls an ein Makromolekül B gebunden ist),

s und t jeweils 0 bis 5 sind,

$R_2$ Wasserstoff, gegebenenfalls substituiertes $C_1$- bis $C_{16}$-Alkyl, Acyl, Acylalkyl (gegebenenfalls substituiert mit einer oder mehreren OH- oder niederen Alkoxygruppen), $-CH_2$-X-V, B oder $CH_2COB$ ist, wobei X CO, gegebenenfalls verzweigtes $C_1$- bis $C_{10}$-Alkylen (gegebenenfalls substituiert mit einer oder mehreren OH- oder niederen Alkoxygruppen) oder gegebenenfalls verzweigtes $C_2$- bis $C_{23}$-Alkylen, unterbrochen durch O, ist,

V $NR^3R^4$ oder $COOR^6$ ist,

$R^3$ und $R^4$ jeweils Wasserstoff, $C_1$- bis $C_{16}$-Alkyl (gegebenenfalls substituiert mit einer oder mehreren OH- oder niederen Alkylgruppen) sind oder zusammen einen 5- bis 6-gliedrigen Heterozyklus bilden, der gegebenenfalls ein weiteres Heteroatom enthält,

$R^6$ Wasserstoff, $C_1$- bis $C_{16}$-, gesättigte, ungesättigte, lineare, verzweigte oder cyclische Kohlenwasserstoff-, Aryl- oder Aralkyl-Gruppe ist,

$R_2$ oder $R_3$ durch eine $C_2$- bis $C_{20}$-Alkylenkette (die gegebenenfalls eine terminale Carbonylgruppe enthält, welche gegebenenfalls durch eine oder mehrere O- oder $R^1$-Carboxymethylimino unterbrochen sind oder mit einer oder mehreren OH-, niederen Alkoxy- oder Carboxy-niederen Alkyl-Gruppen substituiert sind) an ein zweites Makromolekül der Formel

D'

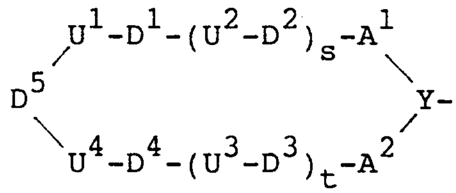

gebunden ist, welches gleich dem Makromolekül D oder von diesem verschieden sein kann.

7. Zusatzstoff nach Anspruch 1, bei dem L und L' jeweils unabhängig voneinander ausgewählt sind aus 1,4,7,10-Tetraazacyclododecan-1,4,7-triessigsäure, 1,4,7-Tris-(carboxymethyl)-10-(2'-hydroxypropyl)-1,4,7,10-tetraazacyclododecan, N,N-Bis[2-[bis(carboxymethyl)-amino]ethyl]glycin, DTPA-bis-methylamid, DTPA-bis-morpholinoamid und DTPA-bis-1,2-dihydroxypropylamid.

8. Zusatzstoff nach Anspruch 1, bei dem L und L' der gleiche organische Ligand sind.

9. Verfahren zur Herstellung einer Kontrastmittelzusammensetzung zur Verwendung bei der bildgebenden magnetischen Resonanz-, Röntgen-, Ultraschall- und Radiodiagnostik-Untersuchung, bei dem
ein Metallion M, das mit einem organischen Ligand L komplexiert ist, und einen Komplexsalz-Zusatzstoff der Formel

$$X_m[X'(L')]_n$$

gemäß einem der Ansprüche 1 bis 8 sowie
ein pharmazeutisch akzeptablen Träger dafür kombiniert werden.

10. Verfahren nach Anspruch 9, bei dem das Molverhältnis des Komplexsalzes zu Metallchelat-Kontrastmittel zwischen etwa 0,05 und 10% liegt.

11. Verfahren nach Anspruch 9, bei dem das Metallion ausgewählt ist aus paramagnetischen Metallatomen, Elementen der Lanthanidenreihe, Yttrium und den Elementen der Übergangsreihe.

12. Verfahren nach Anspruch 11, bei dem die paramagnetischen Metalle ausgewählt sind aus Gadolinium(III), Dysprosium(III), Mangan(II), Mangan(III), Chrom(III), Eisen(II) und Eisen(III).

13. Verfahren nach Anspruch 9, bei dem das mit einem organischen Liganden komplexierte Metallion Gadolinium(III)-1,4,7-tris-(carboxymethyl)-10-(2'-hydroxypropyl)-1,4,7,10-tetraazacyclododecan und der Zusatzstoff Calcium-bis[1,4,7-tris(carboxymethyl)-10-(2'-hydroxypropyl)-1,4,7,10-tetraazacyclododecanatocalcium(II)] ist,
N-Methylglucamin-gadolinium(III)-1,4,7,10-tetraazacyclododecan-N,N',N",N"'-tetraessigsäure und der Zusatzstoff Calcium-[1,4,7,10-tetraazacyclododecan-1,4,7,19-tetraacetatocalcium(II)] ist,
Di-N-methylglucaminium-gadolinium(III)-N,N-bis[2-[bis(carboxymethyl)-amino]ethyl]glycin und der Zusatzstoff Calcium-bis[diethylentriamin-N,N',N',N",N"-pentacetatocalcium(II)] ist,
Diethylentriaminpentaacetato-bis-methylamid-gadolinium(III) und der Zusatzstoff Calcium-bis[diethylentriamin-N,N',N',N",N"-pentaacetato-bis-methylamid-calcium(II)] ist,
Gadolinium(III)-1,4,7,10-tetraazacyclododecan-1,4,7-triessigsäure und der Zusatzstoff Calcium-bis[1,4,7,10-tetraazacyclododecan-1,4,7-triacetatocalcium(II)] ist,
Gadolinium(III)-DTPA-bis-morpholinoamid und der Zusatzstoff Calcium-bis-[DTPA-bis-morpholinamido-calcium(II)] ist oder
Gadolinium(III)-DTPA-bis-1,2-dihydroxypropylamid und der Zusatzstoff Calcium-bis[DTPA-bis-1,2-dihydroxypropylamidocalcium(II)] ist.

14. Verfahren zur Herstellung einer Kontrastmittelzusammensetzung, bei dem
ein Metallchelat, das Gadolinium(III)-1,4,7-tris(carboxymethyl)-10-(2'-hydroxypropyl)-1,4,7,10-tetraazacyclododecan ist,
einen Zusatzstoff, der Calcium-bis[1,4,7-tris(carboxymethyl)-10-(2'-hydroxypropyl)-1,4,7,10-tetraazacyclododecanatocalcium(II)] ist,
einen Puffer,
ausreichend saure und/oder basische Lösung, um den pH-Wert der Zusammensetzung auf einen gewünschten Wert einzustellen und
Wasser kombiniert werden.

**Patentansprüche für folgenden Vertragsstaat : GR**

**1.** Zusatzstoff für ein Metallchelat-Kontrastmittel, bei dem das Metallchelat-Kontrastmittel M(L) ein Metallion umfaßt, das mit einem organischen Ligand komplexiert ist, und der Zusatzstoff die Formel I

$$X_m[X'(L')]_n \quad (I)$$

aufweist, in der X und X' jeweils unabhängig voneinander aus Calcium oder Zink ausgewählt sind, L' ein organischer Ligand ist, der L oder ein anderer organischer Ligand mit einer größeren Affinität zu M als zu Calcium oder Zink sein kann, und in der m und n jeweils unabhängig voneinander 1, 2 oder 3, sind.

**2.** Zusatzstoff nach Anspruch 1, bei dem X = X' = Calcium ist.

**3.** Zusatzstoff nach Anspruch 1, bei dem L und L' jeweils unabhängig voneinander aus linearen und makrocyclischen Polyaminopolycarbonsäuren und deren Derivaten ausgewählt sind.

**4.** Zusatzstoff nach Anspruch 1, bei dem L und L' jeweils unabhängig voneinander aus Verbindungen der Formel

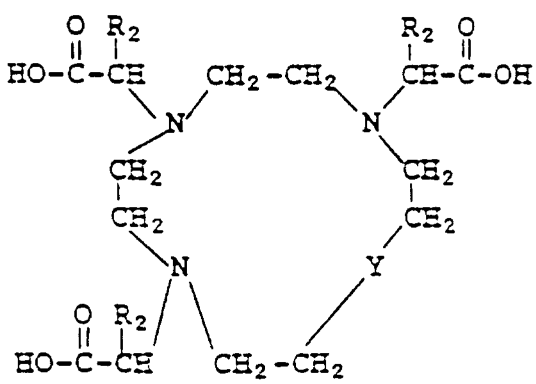

ausgewählt sind, in der
Y Sauerstoff oder

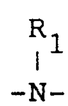

ist,
$R_1$ und $R_2$ jeweils unabhängig Wasserstoff, Alkyl, Arylalkyl, Aryl, Alkoxy, Hydroxyalkyl, Hydroxyalkoxy,

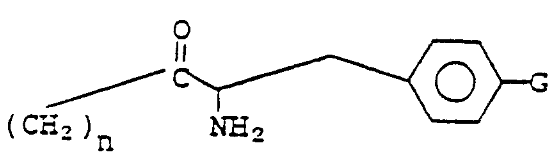

,

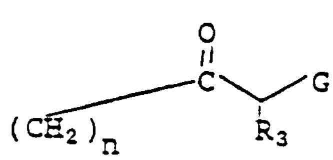

$-(CH_2)_nG$,

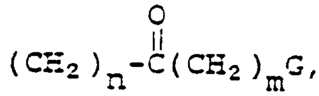 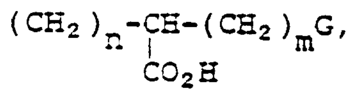

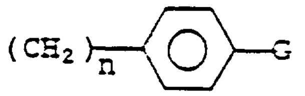 ,

sind, in denen G $NH_2$, NCS,

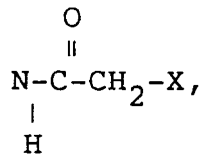

$CO_2H$, $NHR_4$, $N(R_4)_2$, CN ist, wobei $R_4$ Alkyl oder Hydroxyalkyl, Hydroxyalkoxy,

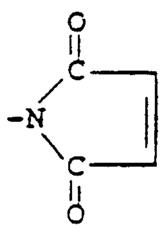 , 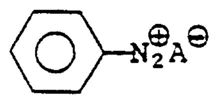

(in der A ein Anion ist),

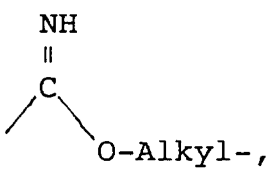 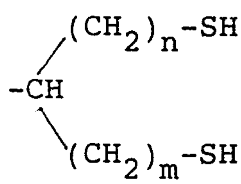

ist, wobei n und m 0 oder eine ganze Zahl von 1 bis 5, $R_3$ Wasserstoff, Hydroxyalkyl, Alkoxy, Alkyl, Aryl, Arylalkyl oder Hydroxyalkoxy und X Chlor, Brom oder Iod ist.

**5.** Zusatzstoff nach Anspruch 1, bei dem L und L' jeweils unabhängig voneinander ausgewählt sind aus Verbindungen der Formel

B

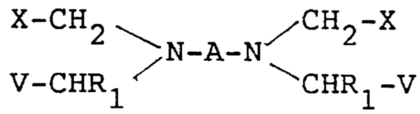

oder

C

$N(CH_2X)_3$,

in denen

X -COOY, $PO_3HY$ oder -CONHOY ist,

Y ein Wasserstoffatom, ein Metallion-Äquivalent und/oder ein physiologisch biokompatibles Kation einer anorganischen oder organischen Base oder Aminosäure ist,

A $-CHR_2-CHR_3-$, $-CH_2CH_2(ZCH_2-CH_2)_m-$,

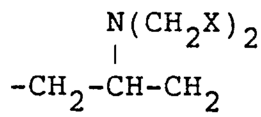 oder 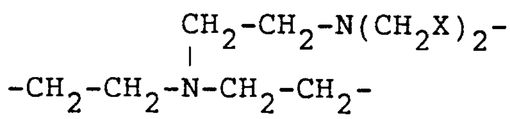

ist, wobei

X wie oben definiert ist,

jedes $R_1$ Wasserstoff oder Methyl ist,

$R_2$ und $R_3$ zusammen eine Trimethylengruppe oder eine Tetramethylengruppe darstellen oder unabhängig voneinander Wasserstoffatome, niedere Alkylgruppen (z.B. 1 bis 8-Kohlenwasserstoffe), Phenylgruppen, Benzylgruppen oder $R_2$ ein Wasserstoffatom ist und $R_3$ $-(CH_2)_p-C_6H_4-W$-Protein ist, wobei p 0 oder 1, W -NH-, $-NHCOCH_2-$ oder -NHCS- ist, Protein ein Proteinrest darstellt,

m 1, 2 oder 3 ist,

Z ein Sauerstoffatom oder ein Schwefelatom oder die Gruppe $NCH_2X$ oder $NCH_2CH_2OR_4$ ist, wobei X wie oben definiert ist und $R_4$ ein $C_1$- bis $C_8$-Alkyl ist,

V X oder $-CH_2OH$, $-CONH(CH_2)_nX$ oder -COB ist, wobei X wie oben definiert ist, B ein Protein- oder Lipidrest ist, n eine ganze Zahl von 1 bis 12 ist, oder, wenn $R_1$, $R_2$ und $R_3$ jeweils Wasserstoff sind, beide V's zusammen die Gruppe

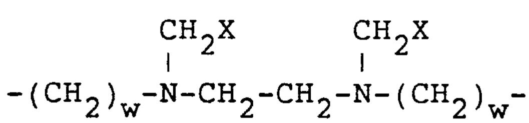

bilden,

in der X wie oben ist, w 1, 2 oder 3 ist, mit der Maßgabe, daß mindestens zwei der Substituenten Y Metallionen-Äquivalente eines Elements mit einer Ordnungszahl von 21 bis 29, 42, 44 oder 57 bis 83 darstellen.

**6.** Zusatzstoff nach Anspruch 1, bei dem L und L' jeweils unabhängig voneinander ausgewählt sind aus Verbindungen der Formel

D

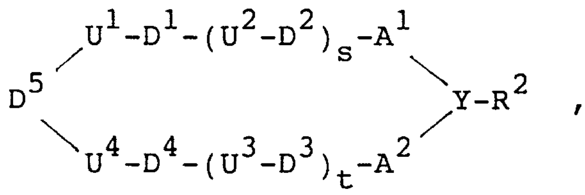

in der

Y N oder P ist,

$A^1$ und $A^2$ jeweils gegebenenfalls verzweigtes $C_2$- bis $C_6$-Alkylen sind,

$U^1$, $U^2$, $U^3$ und $U^4$ jeweils einfach gebundenes oder gegebenenfalls verzweigtes $C_1$- bis $C_6$-Alkylen sind,

$D^1$, $D^2$, $D^3$, $D^4$ jeweils O, S, $C_1$- bis $C_6$-Alkylen oder $NR_7$ ist,

$R_7$ Wasserstoff oder $C_1$- bis $C_4$-Alkylen mit einer $COOR^1$-Endgruppe ist,

$R^1$ Wasserstoff oder ein Metallion-Äquivalent ist,

$^{D5}$ $D^1$ oder $CHR^5$ ist, wobei $R^5$ Wasserstoff oder gegebenenfalls ungesättigtes $C_1$- bis $C_{20}$-Alkylen sein kann, das Imino, Phenylenoxy, Phenylenimino, Amido, Ester, O, S und/oder N, gegebenenfalls substituiert mit OH, SH, Imino und/oder Amino ist, und eine funktionelle Endgruppe tragen kann (die gegebenenfalls an ein Makromolekül B gebunden ist),

s und t jeweils 0 bis 5 sind,

$R_2$ Wasserstoff, gegebenenfalls substituiertes $C_1$- bis $C_{16}$-Alkyl, Acyl, Acylalkyl (gegebenenfalls substituiert mit einer oder mehreren OH- oder niederen Alkoxygruppen), $-CH_2$-X-V, B oder $CH_2COB$ ist, wobei X CO, gegebenenfalls verzweigtes $C_1$- bis $C_{10}$-Alkylen (gegebenenfalls substituiert mit einer oder mehreren OH- oder niederen Alkoxygruppen) oder gegebenenfalls verzweigtes $C_2$- bis $C_{23}$-Alkylen, unterbrochen durch O, ist,

V $NR^3R^4$ oder $COOR^6$ ist,

$R^3$ und $R^4$ jeweils Wasserstoff, $C_1$- bis $C_{16}$-Alkyl (gegebenenfalls substituiert mit einer oder mehreren OH- oder niederen Alkylgruppen) sind oder zusammen einen 5- bis 6-gliedrigen Heterozyklus bilden, der gegebenenfalls ein weiteres Heteroatom enthält,

$R^6$ Wasserstoff, $C_1$- bis $C_{16}$-, gesättigte, ungesättigte, lineare, verzweigte oder cyclische Kohlenwasserstoff-, Aryl- oder Aralkyl-Gruppe ist,

$R_2$ oder $R_3$ durch eine $C_2$- bis $C_{20}$-Alkylenkette (die gegebenenfalls eine terminale Carbonylgruppe enthält, welche gegebenenfalls durch eine oder mehrere O- oder $R^1$-Carboxymethylimino unterbrochen sind oder mit einer oder mehreren OH-, niederen Alkoxy- oder Carboxy-niederen Alkyl-Gruppen substituiert sind) an ein zweites Makromolekül der Formel

D'

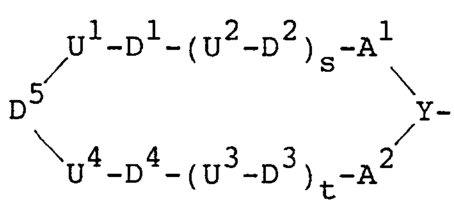

gebunden ist, welches gleich dem Makromolekül D oder von diesem verschieden sein kann.

7. Zusatzstoff nach Anspruch 1, bei dem L und L' jeweils unabhängig voneinander ausgewählt sind aus 1,4,7,10-Tetraazacyclododecan-1,4,7-triessigsäure, 1,4,7-Tris-(carboxymethyl)-10-(2'-hydroxypropyl)-1,4,7,10-tetraazacyclododecan, N,N-Bis[2-[bis(carboxymethyl)-amino]ethyl]glycin, DTPA-bis-methylamid, DTPA-bis-morpholinoamid und DTPA-bis-1,2-dihydroxypropylamid.

8. Zusatzstoff nach Anspruch 1, bei dem L und L' der gleiche organische Ligand sind.

9. Verfahren zur Herstellung einer Kontrastmittelzusammensetzung zur Verwendung bei der bildgebenden magnetischen Resonanz-, Röntgen-, Ultraschall- und Radiodiagnostik-Untersuchung, bei dem
ein Metallion M, das mit einem organischen Ligand L komplexiert ist, und einen Komplexsalz-Zusatzstoff der Formel

$$X_m[X'(L')]_n$$

gemäß einem der Ansprüche 1 bis 8 sowie
ein pharmazeutisch akzeptablen Träger dafür kombiniert werden.

10. Verfahren nach Anspruch 9, bei dem das Molverhältnis des Komplexsalzes zu Metallchelat-Kontrastmittel zwischen etwa 0,05 und 10% liegt.

11. Verfahren nach Anspruch 9, bei dem das Metallion ausgewählt ist aus paramagnetischen Metallatomen, Elementen der Lanthanidenreihe, Yttrium und den Elementen der Übergangsreihe.

12. Verfahren nach Anspruch 11, bei dem die paramagnetischen Metalle ausgewählt sind aus Gadolinium(III), Dysprosium(III), Mangan(II), Mangan(III), Chrom(III), Eisen(II) und Eisen(III).

13. Verfahren nach Anspruch 9, bei dem das mit einem organischen Liganden komplexierte Metallion Gadolinium(III)-1,4,7-tris-(carboxymethyl)-10-(2'-hydroxypropyl)-1,4,7,10-tetraazacyclododecan und der Zusatzstoff Calcium-bis[1,4,7-tris(carboxymethyl)-10-(2'-hydroxypropyl)-1,4,7,10-tetraazacyclododecanatocalcium(II)] ist,
N-Methylglucamin-gadolinium(III)-1,4,7,10-tetraazacyclododecan-N,N',N'',N'''-tetraessigsäure und der Zusatzstoff Calcium-[1,4,7,10-tetraazacyclododecan-1,4,7,19-tetraacetatocalcium(II)] ist,
Di-N-methylglucaminium-gadolinium(III)-N,N-bis[2-[bis(carboxymethyl)-amino]ethyl]glycin und der Zusatzstoff Calcium-bis[diethylentriamin-N,N',N',N'',N''-pentacetatocalcium(II)] ist,
Diethylentriaminpentaacetato-bis-methylamid-gadolinium(III) und der Zusatzstoff Calcium-bis[diethylentriamin-N,N',N',N'',N''-pentaacetato-bis-methylamidcalcium(II)] ist,
Gadolinium(III)-1,4,7,10-tetraazacyclododecan-1,4,7-triessigsäure und der Zusatzstoff Calcium-bis[1,4,7,10-tetraazacyclododecan-1,4,7-triacetatocalcium(II)] ist,
Gadolinium(III)-DTPA-bis-morpholinoamid und der Zusatzstoff Calcium-bis-[DTPA-bis-morpholinamido-calcium(II)] ist oder
Gadolinium(III)-DTPA-bis-1,2-dihydroxypropylamid und der Zusatzstoff Calcium-bis[DTPA-bis-1,2-dihydroxypropylamidocalcium(II)] ist.

14. Verfahren zur Herstellung einer Kontrastmittelzusammensetzung, bei dem
ein Metallchelat, das Gadolinium(III)-1,4,7-tris(carboxymethyl)-10-(2'-hydroxypropyl)-1,4,7,10-tetraazacyclododecan ist,
einen Zusatzstoff, der Calcium-bis(1,4,7-tris(carboxymethyl)-10-(2'-hydroxypropyl)-1,4,7,10-tetraazacyclododecanatocalcium(II)] ist,
einen Puffer,
ausreichend saure und/oder basische Lösung, um den pH-Wert der Zusammensetzung auf einen gewünschten Wert einzustellen und
Wasser kombiniert werden.

## Revendications

### Revendications pour les Etats contractants suivants : AT, BE, CH, DE, DK, FR, GB, IT, LI, LU, NL, SE

1. Un excipient destiné à un agent de contraste contenant un chélate métallique, suivant lequel ledit agent de contraste comportant un chélate métallique M(L) comprend un ion de métal complexé par un ligand organique, ledit excipient ayant la formule

$$X_m[X'(L')]_n \qquad (I)$$

dans laquelle X et X' représentent chacun, indépendamment, Ca ou Zn, L' est un ligand organique qui peut être L ou un autre ligand de plus grande affinité pour le métal M que pour Ca ou Zn; et dans laquelle formule, m et n sont indépendamment 1, 2, ou 3.

**2.** L'excipient de la revendication 1, où X = X' = Ca .

**3.** L'excipient de la revendication 1, dans lequel L et L' sont indépedamment choisi parmi les acides polyaminopolycarboxyliques linéaires ou cycliques et leurs dérivés.

**4.** L'excipient de la revendication 1, dans lequel L et L' sont indépendamment choisi parmi les composés de formule

A

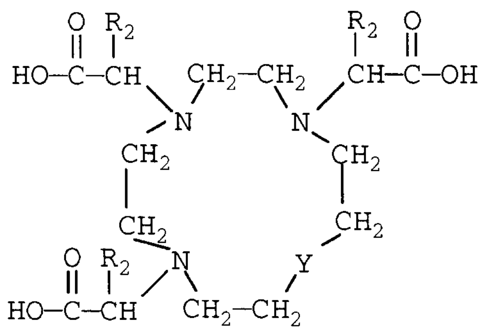

où Y est l'oxygène ou $-NR_1-$; $R_1$ et $R_2$ sont, indépendamment, H, alcoyle, arylalcoyle, aryle, alcoxy, hydroxyalcoyle ou hydroxyalcoxy,

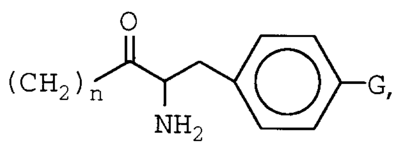 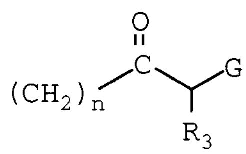 ,

$-(CH_2)_nG$,

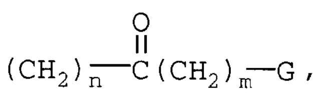 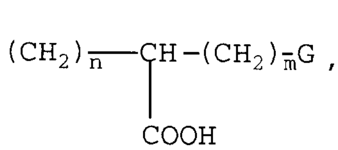 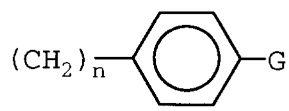

dans lesquels G est $-NH_2$, -NCS, $-NH-CO-CH_2X$, -COOH, $-NHR_4$, $-N(R_4)_2$, -CN, où $R_4$ est un alcoyle ou hydroxyalcoyle, hydroxyalcoxy,

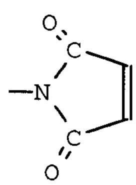 , 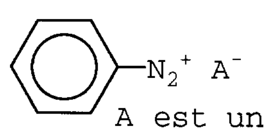 (où A est un anion),

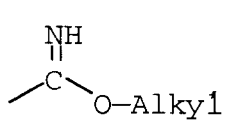 , 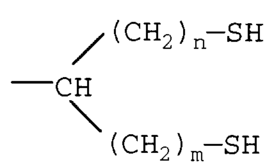 ,

où n et m représentent zéro ou un entier de 1 à 5, $R_3$ est H, hydroxyalcoyle, alcoxy, alcoyle, aryle, arylalcoyle ou hydroxyalcoxy, et X est Cl, Br, ou I.

**5.** L'excipient de la revendication 1, dans lequel L et L' sont indépendamment choisi parmi les composés de formule

B

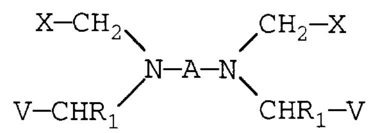

ou

C

$$N(CH_2X)_3,$$

dans lesquels X est -COOH, -OPOOY ou -CONHOY;

Y est H, un équivalent d'ion métallique et/ou un cation physiologiquement biocompatible provenant d'une base ou d'un acide aminé;

A est choisi parmi $-CHR_2-CHR_3-$, $-(CH_2)_2(ZCH_2-CH_2)_m-$,

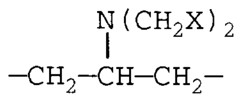 , or 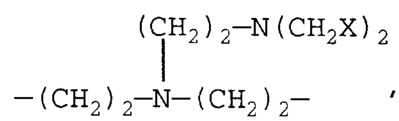 ,

où X est défini comme ci-dessus; chaque $R_1$ est H ou $-CH_3$;

$R_2$ et $R_3$ représentent ensemble un groupe triméthylène ou tetraméthylène ou, pris individuellement, représentent les restes suivants: H, alcoyles inférieurs (p. ex. à $C_{1-8}$), phényles benzyles; ou $R_2$ est H et $R_3$ est un reste $-(CH_2)_p-C_6H_4-W$-protéine où p est 0 ou 1, W est -NH-, $-NHCOCH_2-$ ou -NHCS-, "protéine" représente un reste protéine; m est 1, 2 ou 3;

Z est O ou S ou le groupe $-NCH_2X$ ou $-N(CH_2)_2OR_4$, où X est défini comme plus haut et $R_4$ est un alcoyle en $C_{1-8}$;

V est X, ou $-CH_2OH$, $-CONH(CH_2)_nX$ ou -COB où X est défini comme ci-dessus, B est une protéine ou un résidu lipidique, n est un entier de 1 à 12 ou, si $R_1$, $R_2$, et $R_3$ sont tous H, alors les deux V forment ensemble le groupe

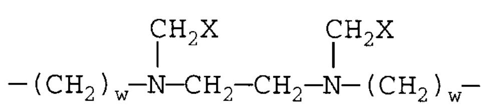

où X est comme ci-dessus, w est 1, 2, ou 3, à condition qu'au moins deux des substituants Y représentent des équivalents ioniques d'éléments de nombres atomiques 21-29, 42, 44, ou 57-83.

6. L'excipient de la revendication 1, dans lequel L et L' sont indépendamment choisi parmi les composés de formule

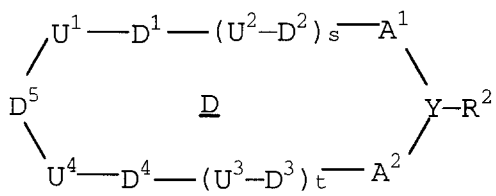

où Y est N ou P;

$A^1$ et $A^2$ sont des alcoylènes en $C_{2-6}$, facultativement ramifiés;

$U^1$, $U^2$, $U^3$, et $U^4$ sont chacun une liaison simple ou un alcoylène en $C_{1-6}$;

$D^1$, $D^2$, $D^3$, et $D^4$ sont chacun O, S, un alcoylène en $C_{1-6}$ ou le groupe $-NR_7$;

$R_7$ est H ou un alcoylène en $C_{1-4}$ comportant un groupe teminal $-COOR^1$;

$R^1$ est H ou un équivalent ionique d'un métal;

$D^5$ est $D^1$ ou $CHR^5$ où $R^5$ peut être H ou un alcoylène facultativement insaturé en $C_{1-20}$ pouvant inclure des fonctions imino, phénylèneoxy, phénylèneimino, amido, ester, O, S, et/ou N, elles-même facultativement substituées par -OH, -SH, imino et/ou amino, et pouvant comporter un groupe terminal fonctionnel (facultativement lié à une macromolécule B);

s et t peuvent valoir chacun de 0 à 5;

$R^2$ est H, un alcoyle en $C_{1-16}$ facultativement substitué, acyle, acylalcoyle (facultativement substitué par un ou plusieurs -OH ou groupes alcoyloxy inférieurs), $-CH_2$-X-V, B, ou $-CH_2COB$, où X est -CO-, un alcoylène en $C_{1-10}$, (facultativement ramifié et facultativement substitué par un ou plusieurs -OH ou groupes alcoyloxy inférieurs), ou un alcoylène en $C_{2-23}$, facultativement ramifié et interrompu par un -O-;

V est $-NR^3R^4$ ou $-COOR^6$; $R^3$ et $R^4$ sont chacun H, un alcoyle en $C_{1-16}$, (facultativement substitué par un ou plusieurs OH ou alcoyloxy inférieurs), ou, pris ensemble, forment un hétérocycle contenant facultativement un autre hétéroatome;

$R^6$ est H, un radical hydrocarburé saturé ou non, linéaire, ramifié ou cyclique, un groupe aryle ou aralcoyle;

$R^2$ ou $R^3$ peuvent être liés par une chaîne alcoylénique en $C_{2-20}$ (comportant facultativement un carbonyle terminal et facultativement interrompue par un ou plusieurs O ou $R^1$, carboxyméthylimino, ou substituée par un ou plusieurs OH, alcoyloxy inférieurs ou alcoyles inférieurs carboxylés) à une deuxième macromolécule D' de formule

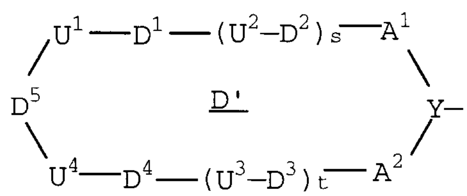

laquelle peut être la même que la macromolécule D ou différente de celle-ci.

7. L'excipient de la revendication 1, dans lequel L et L' sont indépendamment choisi parmi l'acide 1,4,7,10-tetraazacyclododecane-1,4,7-triacétique, le 1,4,7-tris(carboxyméthyl)-10-(2'hydroxypropyl)-1,4,7,10-tetraazacyclododecane, la N,N-bis[2-[bis(carboxyméthyl)amino]éthyl]glycine, le DTPA-bisméthylamide, le DTPA-bismorpholide, et le DTPA-bis-1,2-dihydroxypropylamide.

8. L'excipient de la revendication 1, dans lequel L et L' sont le même ligand organique.

9. Composition d'agent de contraste utilisable en imagerie par résonance magnétique, rayons X, ultrasons, et radiodiagnostique, comportant un ion de métal complexé par un ligand organique L et un excipient sous forme de sel complexe de formule

$$X_m[X'(L')]_n \qquad (I)$$

suivant l'une des revendications 1 à 8, et une phase porteuse pharmaceutiquement acceptable.

10. La composition de la revendication 9, où le rapport molaire dudit sel complexe audit agent de contraste sous forme de chélate métallique est d'environ 0,05 à 10%.

11. La composition de la revendication 9, dans laquelle ledit ion de métal est choisi parmi les atomes de métaux paramagnétiques, les éléments de la série des lanthanides, l'yttrium, et les éléments de la série de transition.

12. La composition de la revendication 9, dans laquelles les métaux paramagnétiques sont choisis parmi gadolinium$^{III}$, dysprosium$^{III}$, manganèse$^{III}$, manganèse$^{III}$, chrome$^{III}$, fer$^{II}$ et fer$^{III}$.

13. La composition de la revendication 9, dans laquelle ledit ion de métal complexé par un ligand organique est le Gd$^{III}$-1,4,7-tris(carboxyméthyl)-10-(2'hydroxypropyl)-1,4,7, 10-tetraazacyclododecane, et ledit excipient est le Ca-bis[1,4,7-tris(carboxyméthyl)-10-(2'hydroxypropyl)-1,4,7, 10-tetraazacyclododecanatocalcium$^{II}$];
le 1,4,7,10-tetraazacyclododecane-N,N',N",N"'-tetraacétate de N-méthylglucamine-Gd$^{III}$, et ledit excipient est le Ca-[1,4,7,10-tetraazacyclododecane-1,4,7,10-tetraacétato-calcium$^{II}$];
le N-méthylglucaminium-gadolinium$^{III}$-N,N-bis[2-[bis(carboxyméthyl)amino]éthyl]glycine et ledit excipient est le calcium-bis[diéthylènetriamino-N,N',N",N"'-pentaacétatocalcium$^{II}$];
le diéthylènetriamino-pentaacétato-bisméthylamidogadolinium$^{III}$, et ledit excipient est le calcium-bis[diéthylènetriamino-N,N',N",N"'-pentaacétatobisméthylamidocalcium$^{II}$];
le 1,4,7,10-tetraazacyclododecane-1,4,7,-triacétate de gadolinium$^{III}$ et ledit excipient est le calcium-bis[1,4,7,10-tetraazacyclododecane-1,4,7,-triacétatocalcium$^{II}$];
le gadolinium$^{III}$-DTPA-bis-morpholide et ledit excipient est le calcium-bis[DTPA-bis-morpholidocalcium$^{II}$];
le gadolinium$^{III}$-DTPA-bis[1,2-dihydroxypropylamide] et ledit excipient est le calcium-bis[DTPA-bis-1,2-dihydroxypropylamidocalcium$^{II}$].

14. Une composition d'agent de contraste comprenant un chélate d'un métal constitué par le Gd$^{III}$-1,4,7-tris(carboxyméthyl)-10-(2'hydroxypropyl)-1,4,7,10-tetraazacyclododecane; un excipient constitué par le le Ca-bis[1,4,7-tris(carboxyméthyl)-10-(2'hydroxypropyl)-1,4,7,10-tetraazacyclododecanatocalcium$^{II}$]; un tampon; une quantité de solution acide ou basique suffisante pour ajuster le pH de la composition à une valeur voulue; et de l'eau.

**Revendications pour l'Etat contractant suivant : ES**

1. Procédé pour préparer un excipient destiné à un agent de contraste contenant un chélate métallique M(L) comprenant un ion de métal complexé par un ligand organique, ledit excipient ayant la formule

$$X_m[X'(L')]_n \qquad (I)$$

dans laquelle X et X' représentent chacun, indépendamment, Ca ou Zn, L' est un ligand organique qui peut être L ou un autre ligand de plus grande affinité pour le métal M que pour Ca ou Zn; et dans laquelle formule m et n sont indépendamment 1, 2, ou 3, caractérisé en ce qu'on fait réagir le ligand L' avec un sel de Ca ou de Zn..

2. Le procédé de la revendication 1 pour préparer un excipient de formule I, où X = X' = Ca .

3. Le procédé de la revendication 1 pour préparer un excipient de formule I, dans lequel L et L' sont indépedamment choisi parmi les acides polyaminopolycarboxyliques linéaires ou cycliques et leurs dérivés.

4. Le procédé de la revendication 1 pour préparer un excipient de formule I, dans lequel L et L' sont indépedamment choisi parmi les composés de formule

A

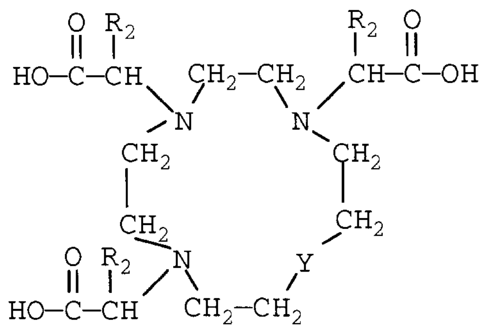

où Y est l'oxygène ou $-NR_1-$; $R_1$ et $R_2$ sont, indépendamment, H, alcoyle, arylalcoyle, aryle, alcoxy, hydroxyalcoyle ou hydroxyalcoxy,

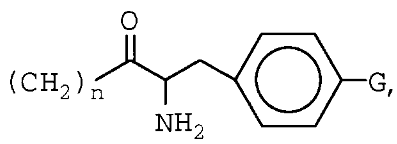 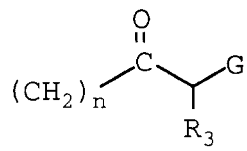 ,

$-(CH_2)_nG$,

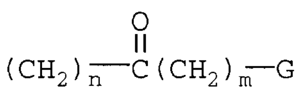 , 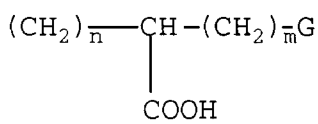 , 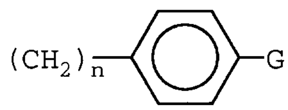

dans lesquels G est $-NH_2$, -NCS, $-NH-CO-CH_2X$, -COOH, $-NHR_4$, $-N(R_4)_2$, -CN, où $R_4$ est un alcoyle ou hydroxyalcoyle, hydroxyalcoxy,

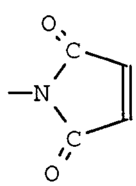 , 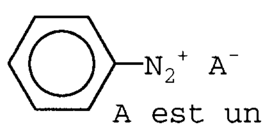 (où A est un anion),

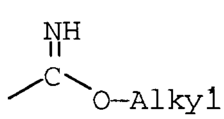 , 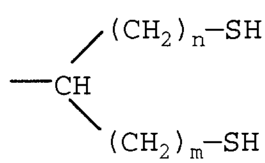 ,

où n et m représentent zéro ou un entier de 1 à 5, $R_3$ est H, hydroxyalcoyle, alcoxy, alcoyle, aryle, arylalcoyle ou hydroxyalcoxy, et X est Cl, Br, ou I.

5. Le procédé de la revendication 1 pour préparer un excipient de formule I, dans lequel L et L' sont indépendamment choisi parmi les composés de formule

B

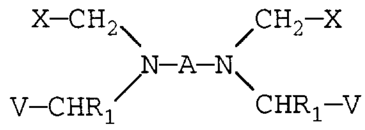

ou

C

$$N(CH_2X)_3,$$

dans lesquels X est -COOH, -OPOOY ou -CONHOY;

Y est H, un équivalent d'ion métallique et/ou un cation physiologiquement biocompatible provenant d'une base ou d'un acide aminé;

A est choisi parmi $-CHR_2-CHR_3-$, $-(CH_2)_2(ZCH_2-CH_2)_m-$,

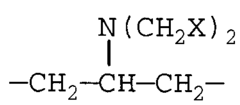 , or 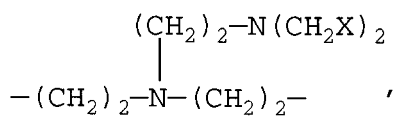 ,

où X est défini comme ci-dessus; chaque $R_1$ est H ou $-CH_3$;

$R_2$ et $R_3$ représentent ensemble un groupe triméthylène ou tetraméthylène ou, pris individuellement, représentent les restes suivants: H, alcoyles inférieurs (p. ex. à $C_{1-8}$), phényles benzyles; ou $R_2$ est H et $R_3$ est un reste $-(CH_2)_p-C_6H_4-$W-protéine où p est 0 ou 1, W est -NH-, $-NHCOCH_2-$ ou -NHCS-, "protéine" représente un reste protéine; m est 1, 2 ou 3;

Z est O ou S ou le groupe $-NCH_2X$ ou $-N(CH_2)_2OR_4$, où X est défini comme plus haut et $R_4$ est un alcoyle en $C_{1-8}$;

V est X, ou $-CH_2OH$, $-CONH(CH_2)_nX$ ou -COB où X est défini comme ci-dessus, B est une protéine ou un résidu lipidique, n est un entier de 1 à 12 ou, si $R_1$, $R_2$, et $R_3$ sont tous H, alors les deux V forment ensemble le groupe

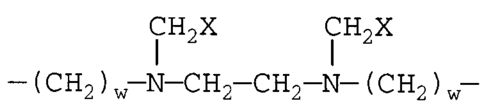

où X est comme ci-dessus, w est 1, 2, ou 3, à condition qu'au moins deux des substituants Y représentent des équivalents ioniques d'éléments de nombres atomiques 21-29, 42, 44, ou 57-83.

6. Le procédé de la revendication 1 pour préparer un excipient de formule I, dans lequel L et L' sont indépedamment choisi parmi les composés de formule

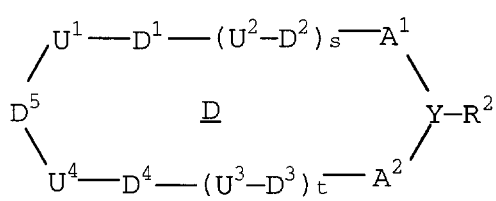

où Y est N ou P;

$A^1$ et $A^2$ sont des alcoylènes en $C_{2-6}$, facultativement ramifiés;

$U^1$, $U^2$, $U^3$, et $U^4$ sont chacun une liaison simple ou un alcoylène en $C_{1-6}$;

$D^1$, $D^2$, $D^3$, et $D^4$ sont chacun O, S, un alcoylène en $C_{1-6}$ ou le groupe $-NR_7$;

$R_7$ est H ou un alcoylène en $C_{1-4}$ comportant un groupe teminal $-COOR^1$;

$R^1$ est H ou un équivalent ionique d'un métal;

$D^5$ est $D^1$ ou $CHR^5$ où $R^5$ peut être H ou un alcoylène facultativement insaturé en $C_{1-20}$ pouvant inclure des fonctions imino, phénylèneoxy, phénylèneimino, amido, ester, O, S, et/ou N, elles-même facultativement substituées par -OH, -SH, imino et/ou amino, et pouvant comporter un groupe terminal fonctionnel (facultativement lié à une macromolécule B);

s et t peuvent valoir chacun de 0 à 5;

$R^2$ est H, un alcoyle en $C_{1-16}$ facultativement substitué, acyle, acylalcoyle (facultativement substitué par un ou plusieurs -OH ou groupes alcoyloxy inférieurs), $-CH_2-X-V$, B, ou $-CH_2COB$, où X est -CO-, un alcoylène en $C_{1-10}$, (facultativement ramifié et facultativement substitué par un ou plusieurs -OH ou groupes alcoyloxy inférieurs), ou un alcoylène en $C_{2-23}$, facultativement ramifié et interrompu par un -O-;

V est $-NR^3R^4$ ou $-COOR^6$; $R^3$ et $R^4$ sont chacun H, un alcoyle en $C_{1-16}$, (facultativement substitué par un ou plusieurs OH ou alcoyloxy inférieurs), ou, pris ensemble, forment un hétérocycle contenant facultativement un autre hétéroatome;

$R^6$ est H, un radical hydrocarburé saturé ou non, linéaire, ramifié ou cyclique, un groupe aryle ou aralcoyle;

$R^2$ ou $R^3$ peuvent être liés par une chaîne alcoylénique en $C_{2-20}$ (comportant facultativement un carbonyle terminal et facultativement interrompue par un ou plusieurs O ou $R^1$, carboxyméthylimino, ou substituée par un ou plusieurs OH, alcoyloxy inférieurs ou alcoyles inférieurs carboxylés) à une deuxième macromolécule D' de formule

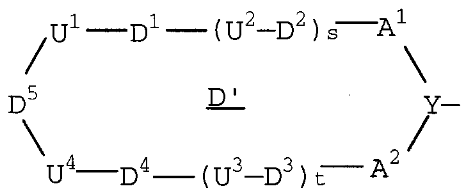

laquelle peut être la même que la macromolécule D ou différente de celle-ci.

7. Le procédé de la revendication 1 pour préparer un excipient de formule I, dans lequel L et L' sont indépendamment choisi parmi l'acide 1,4,7,10-tetraazacyclododecane-1,4,7-triacétique, le 1,4,7-tris(carboxyméthyl)-10-(2'hydroxypropyl)-1,4,7,10-tetraazacyclododecane, la N,N-bis[2-[bis(carboxyméthyl)amino]éthyl]glycine, le DTPA-bisméthylamide, le DTPA-bismorpholide, et le DTPA-bis-1,2-dihydroxypropylamide.

8. Le procédé de la revendication 1 pour préparer un excipient de formule I, dans lequel L et L' sont le même ligand organique.

9. Procédé pour préparer une composition d'agent de contraste utilisable en imagerie par résonance magnétique, rayons X, ultrasons, et radiodiagnostique, suivant lequel on combine un ion de métal M complexé par un ligand organique L et un excipient sous forme de sel complexe de formule

$$X_m[X'(L')]_n \qquad (I)$$

suivant l'une des revendications 1 à 8, et une phase porteuse pharmaceutiquement acceptable.

10. Le procédé de la revendication 9, où le rapport molaire dudit sel complexe audit agent de contraste sous forme de chélate métallique est d'environ 0,05 à 10%.

11. Le procédé de la revendication 9, dans lequel ledit ion de métal est choisi parmi les atomes de métaux paramagnétiques, les éléments de la série des lanthanides, l'yttrium, et les éléments de la série de transition.

12. Le procédé de la revendication 11, dans lequel les métaux paramagnétiques sont choisis parmi gado(inium$^{III}$, dysprosium$^{III}$, manganèse$^{II}$, manganèse$^{III}$, chrome$^{III}$, fer$^{II}$ et fer$^{III}$.

13. Le procédé de la revendication 9, dans lequel ledit ion de métal complexé par un ligand organique est le Gd$^{III}$-1,4,7-tris(carboxyméthyl)-10-(2'hydroxypropyl)-1,4,7,10- tetraazacyclododecane, et ledit excipient est le Ca-bis[1,4,7-tris(carboxyméthyl)-10-(2'hydroxypropyl)-1,4,7, 10-tetraazacyclododecanatocalcium$^{II}$];

le 1,4,7,10-tetraazacyclododecane-N,N',N",N'"-tetraacétate de N-méthylglucamine-Gd$^{III}$, et ledit excipient

est le Ca-[1,4,7,10-tetraazacyclododecane-1,4,7,10-tetraacétatocalcium$^{II}$];

le N-méthylglucaminium-gadolinium$^{III}$-N,N-bis[2-[bis(carboxyméthyl)amino]éthyl]glycine et ledit excipient est le calcium-bis[diéthylènetriamino-N,N',N'',N'''-pentaacétatocalcium$^{II}$];

le diéthylènetriamino-pentaacétato-bisméthylamidogadolinium$^{III}$, et ledit excipient est le calcium-bis[diéthylènetriamino-N,N',N'',N'''-pentaacétatobisméthylamidocalcium$^{II}$];

le 1,4,7,10-tetraazacyclododecane-1,4,7,-triacétate de gadolinium$^{III}$ et ledit excipient est le calcium-bis[1,4,7,10-tetraazacyclododecane-1,4,7,-triacétatocalcium$^{II}$];

le gadolinium$^{III}$-DTPA-bis-morpholide et ledit excipient est le calcium-bis[DTPA-bis-morpholidocalcium$^{II}$];

le gadolinium$^{III}$-DTPA-bis[1,2-dihydroxypropylamide] et ledit excipient est le calcium-bis[DTPA-bis-1,2-dihydroxypropylamidocalcium$^{II}$].

**14.** Procédé pour préparer une composition d'agent de contraste suivant lequel on combine un chélate d'un métal constitué par le Gd$^{III}$-1,4,7-tris(carboxyméthyl)-10-(2'-hydroxypropyl)-1,4,7,10-tetraaza-cyclododecane; un excipient constitué par le le Ca-bis[1,4,7-tris(carboxyméthyl)-10-(2'-hydroxypropyl)-1,4,7,10-tetraazacyclododecanatocalcium$^{II}$]; un tampon; une quantité de solution acide ou basique suffisante pour ajuster le pH de la composition à une valeur voulue; et de l'eau.

**Revendications pour l'Etat contractant suivant : GR**

**1.** Un excipient destiné à un agent de contraste contenant un chélate métallique, suivant lequel ledit agent de contraste comportant un chélate métallique M(L) comprend un ion de métal complexé par un ligand organique, ledit excipient ayant la formule

$$X_m[X'(L')]_n \qquad (I)$$

dans laquelle X et X' représentent chacun, indépendamment, Ca ou Zn, L' est un ligand organique qui peut être L ou un autre ligand de plus grande affinité pour le métal M que pour Ca ou Zn; et dans laquelle formule m et n sont indépendamment 1, 2, ou 3.

**2.** L'excipient de la revendication 1, où X = X' = Ca .

**3.** L'excipient de la revendication 1, dans lequel L et L' sont indépedamment choisi parmi les acides polyaminopolycarboxyliques linéaires ou cycliques et leurs dérives.

**4.** L'excipient de la revendication 1, dans lequel L et L' sont indépedamment choisi parmi les composés de formule

<u>A</u>

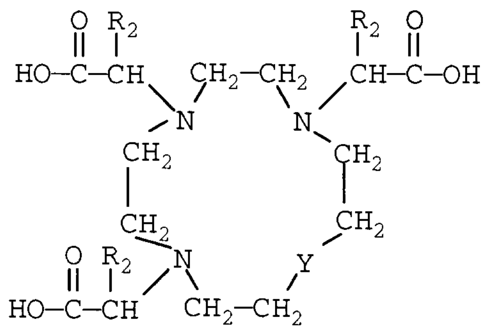

où Y est l'oxygène ou -$NR_1$-; $R_1$ et $R_2$ sont, indépendamment, H, alcoyle, arylalcoyle, aryle, alcoxy, hydroxyalcoyle ou hydroxyalcoxy,

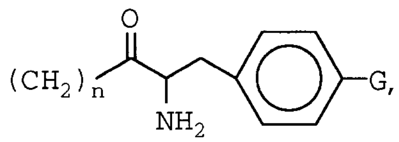

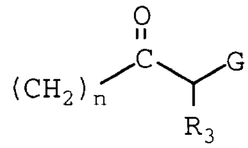

,

$-(CH_2)_nG$,

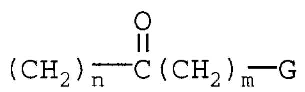

,

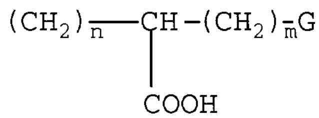

,

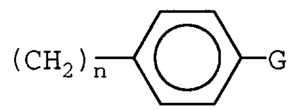

dans lesquels G est $-NH_2$, -NCS, $-NH-CO-CH_2X$, -COOH, $-NHR_4$, $-N(R_4)_2$, -CN, où $R_4$ est un alcoyle ou hydroxyalcoyle, hydroxyalcoxy,

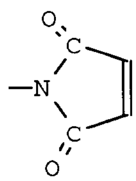

,

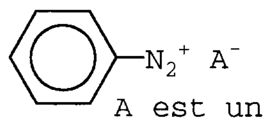

(où A est un anion),

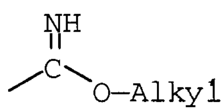

,

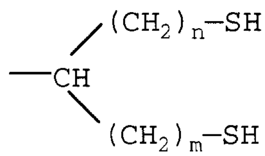

,

où n et m représentent zéro ou un entier de 1 à 5, $R_3$ est H, hydroxyalcoyle, alcoxy, alcoyle, aryle, arylalcoyle ou hydroxyalcoxy, et X est Cl, Br, ou I.

**5.** L'excipient de la revendication 1, dans lequel L et L' sont indépendamment choisi parmi les composés de formule

B

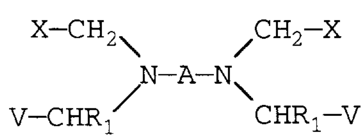

ou

C

$$N(CH_2X)_3,$$

dans lesquels X est -COOH, -OPOOY ou -CONHOY;

Y est H, un équivalent d'ion métallique et/ou un cation physiologiquement biocompatible provenant d'une base ou d'un acide aminé;

A est choisi parmi $-CHR_2-CHR_3-$, $-(CH_2)_2(ZCH_2-CH_2)_m-$,

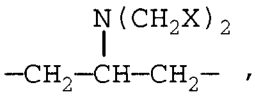 , or 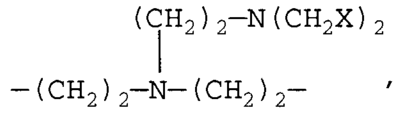 ,

où X est défini comme ci-dessus; chaque $R_1$ est H ou $-CH_3$;

$R_2$ et $R_3$ représentent ensemble un groupe triméthylène ou tetraméthylène ou, pris individuellement, représentent les restes suivants: H, alcoyles inférieurs (p. ex. à $C_{1-8}$), phényles benzyles; ou $R_2$ est H et $R_3$ est un reste $-(CH_2)_p-C_6H_4$-W-protéine où p est 0 ou 1, W est -NH-, $-NHCOCH_2-$ ou -NHCS-, "protéine" représente un reste protéine; m est 1, 2 ou 3;

Z est O ou S ou le groupe $-NCH_2X$ ou $-N(CH_2)_2OR_4$, où X est défini comme plus haut et $R_4$ est un alcoyle en $C_{1-8}$;

V est X, ou $-CH_2OH$, $-CONH(CH_2)_nX$ ou -COB où X est défini comme ci-dessus, B est une protéine ou un résidu lipidique, n est un entier de 1 à 12 ou, si $R_1$, $R_2$, et $R_3$ sont tous H, alors les deux V forment ensemble le groupe

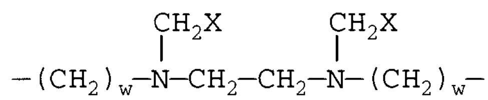

où X est comme ci-dessus, w est 1, 2, ou 3, à condition qu'au moins deux des substituants Y représentent des équivalents ioniques d'éléments de nombres atomiques 21-29, 42, 44, ou 57-83.

**6.** L'excipient de la revendication 1, dans lequel L et L' sont indépendamment choisi parmi les composés de formule

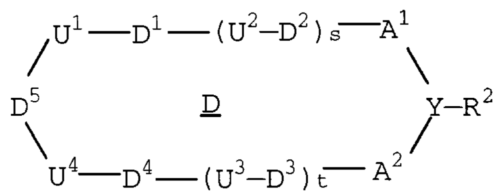

où Y est N ou P;

$A^1$ et $A^2$ sont des alcoylènes en $C_{2-6}$, facultativement ramifiés;

$U^1$, $U^2$, $U^3$, et $U^4$ sont chacun une liaison simple ou un alcoylène en $C_{1-6}$;

$D^1$, $D^2$, $D^3$, et $D^4$ sont chacun O, S, un alcoylène en $C_{1-6}$ ou le groupe $-NR_7$;

$R_7$ est H ou un alcoylène en $C_{1-4}$ comportant un groupe teminal $-COOR^1$;

$R^1$ est H ou un équivalent ionique d'un métal;

$D^5$ est $D^1$ ou $CHR^5$ où $R^5$ peut être H ou un alcoylène facultativement insaturé en $C_{1-20}$ pouvant inclure des fonctions imino, phénylèneoxy, phénylèneimino, amido, ester, O, S, et/ou N, elles-même facultativement substituées par -OH, -SH, imino et/ou amino, et pouvant comporter un groupe terminal fonctionnel (facultativement lié à une macromolécule B);

s et t peuvent valoir chacun de 0 à 5;

$R^2$ est H, un alcoyle en $C_{1-16}$ facultativement substitué, acyle, acylalcoyle (facultativement substitué par un ou plusieurs -OH ou groupes alcoyloxy inférieurs), $-CH_2$-X-V, B, ou $-CH_2COB$, où X est -CO-, un alcoylène en $C_{1-10}$, (facultativement ramifié et facultativement substitué par un ou plusieurs -OH ou groupes alcoyloxy inférieurs), ou un alcoylène en $C_{2-23}$, facultativement ramifié et interrompu par un -O-;

V est $-NR^3R^4$ ou $-COOR^6$; $R^3$ et $R^4$ sont chacun H, un alcoyle en $C_{1-16}$, (facultativement substitué par un ou plusieurs OH ou alcoyloxy inférieurs), ou, pris ensemble, forment un hétérocycle contenant facultativement un autre hétéroatome;

$R^6$ est H, un radical hydrocarburé saturé ou non, linéaire, ramifié ou cyclique, un groupe aryle ou aralcoyle;

$R^2$ ou $R^3$ peuvent être liés par une chaîne alcoylénique en $C_{2-20}$ (comportant facultativement un carbonyle terminal et facultativement interrompue par un ou plusieurs O ou $R^1$, carboxyméthylimino, ou substituée par un ou plusieurs OH, alcoyloxy inférieurs ou alcoyles inférieurs carboxylés) à une deuxième macromolécule D' de formule

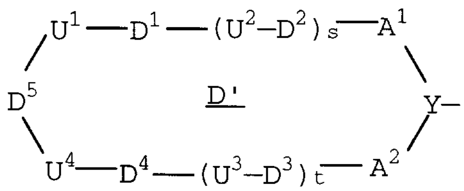

laquelle peut être la même que la macromolécule D ou différente de celle-ci.

7. L'excipient de la revendication 1, dans lequel L et L' sont indépendamment choisi parmi l'acide 1,4,7,10-tetraaza-cyclododecane-1,4,7-triacétique, le 1,4,7-tris(carboxyméthyl)-10-(2'hydroxypropyl)-1,4,7,10-tetraazacyclododecane, la N,N-bis[2-[bis(carboxyméthyl)amino]éthyl]glycine, le DTPA-bisméthylamide, le DTPA-bismorpholide, et le DTPA-bis-1,2-dihydroxypropylamide.

8. L'excipient de la revendication 1, dans lequel L et L' sont le même ligand organique.

9. Procédé pour préparer une composition d'agent de contraste utilisable en imagerie par résonance magnétique, rayons X, ultrasons, et radiodiagnostique, suivant lequel on combine un ion de métal M complexé par un ligand organique L et un excipient sous forme de sel complexe de formule

$$X_m[X'(L')]_n \qquad (I)$$

suivant l'une des revendications 1 à 8, et une phase porteuse pharmaceutiquement acceptable.

10. Le procédé de la revendication 9, où le rapport molaire dudit sel complexe audit agent de contraste sous forme de chélate métallique est d'environ 0,05 à 10%.

11. Le procédé de la revendication 9, dans lequel ledit ion de métal est choisi parmi les atomes de métaux paramagnétiques, les éléments de la série des lanthanides, l'yttrium, et les éléments de la série de transition.

12. Le procédé de la revendication 11, dans lequel les métaux paramagnétiques sont choisis parmi gadolinium$^{III}$, dysprosium$^{III}$, manganèse$^{II}$, manganèse$^{III}$, chrome$^{III}$, fer$^{II}$ et fer$^{III}$.

13. Le procédé de la revendication 9, dans lequel ledit ion de métal complexé par un ligand organique est le Gd$^{III}$-1,4,7-tris(carboxyméthyl)-10-(2'hydroxypropyl)-1,4,7,10-tetraazacyclododecane, et ledit excipient est le Ca-bis[1,4,7-tris(carboxyméthyl)-10-(2'hydroxypropyl)-1,4,7, 10-tetraazacyclododecanatocalcium$^{II}$];
le 1,4,7,10-tetraazacyclododecane-N,N',N'',N'''-tetraacétate de N-méthylglucamine-Gd$^{III}$, et ledit excipient est le Ca-[1,4,7,10-tetraazacyclododecane-1,4,7,10-tetraacétato-calcium$^{II}$];
le N-méthylglucaminium-gadolinium$^{III}$-N,N-bis[2-[bis(carboxyméthyl)amino]éthyl]glycine et ledit excipient est le calcium-bis[diéthylènetriamino-N,N',N'',N'''-pentaacétatocalcium$^{II}$];
le diéthylènetriamino-pentaacétato-bisméthylamidogadolinium$^{III}$, et ledit excipient est le calcium-bis[diéthylènetriamino-N,N',N'',N'''-pentaacétatobisméthylamidocalcium$^{II}$];
le 1,4,7,10-tetraazacyclododecane-1,4,7,-triacétate de gadolinium$^{III}$ et ledit excipient est le calcium-bis[1,4,7,10-tetraazacyclododecane-1,4,7,-triacétatocalcium$^{II}$];
le gadolinium$^{III}$-DTPA-bis-morpholide et ledit excipient est le calcium-bis[DTPA-bis-morpholidocalcium$^{II}$];
le gadolinium$^{III}$-DTPA-bis[1,2-dihydroxypropylamide] et ledit excipient est le calcium-bis[DTPA-bis-1,2-dihydroxypropylamidocalcium$^{II}$].

14. Procédé pour préparer une composition d'agent de contraste suivant lequel on combine un chélate d'un métal constitué par le Gd$^{III}$-1,4,7-tris(carboxyméthyl)-10-(2'-hydroxypropyl)-1,4,7,10-tetraaza-cyclododecane; un excipient constitué par le le Ca-bis[1,4,7-tris(carboxyméthyl)-10-(2'-hydroxypropyl)-1,4,7,10-tetraazacyclododecanatocalcium$^{II}$]; un tampon; une quantité de solution acide ou basique suffisante pour ajuster le pH de la composition à une valeur voulue; et de l'eau.